# EUROPEAN PATENT SPECIFICATION

(11) **EP 0 425 563 B1**
(45) Date of publication and mention of the grant of the patent: **15.05.1996**
(21) Application number: 89908607.8
(22) Date of filing: 19.07.1989
(51) Int. Cl.: C12Q 1/68, C07H 15/12, G01N 33/48

(54) **PROCESS FOR AMPLIFYING AND DETECTING NUCLEIC ACID SEQUENCES**
VERFAHREN ZUR AMPLIFIZIERUNG UND ZUM NACHWEIS VON NUKLEINSÄURESEQUENZEN
PROCEDE D'AMPLIFICATION ET DE DETECTION DE SEQUENCES D'ACIDE NUCLEIQUE

(30) Priority: 20.07.1988 US 221750
(43) Date of publication of application: 08.05.1991
(73) Proprietor: Segev, David, Mazkeret Batya (IL)
(72) Inventor: Segev, David, Mazkeret Batya (IL)
(74) Representative: Grünecker, Kinkeldey, Stockmair & Schwanhäusser Anwaltssozietät
(86) International application number: US8903125
(87) International publication number: WO9001069

(56) References cited:
- EP-A- 0 185 494
- EP-A- 0 246 864
- EP-A- 0 320 308
- EP-A- 0 324 616
- WO-A-89/12696
- US-A- 4 683 195
- US-A- 4 710 465
- DNA vol.7, no.3, April 1988, New York, NY (US); CHANG et al.: "Site-Specific Oligonucleotide-Directed Mutagenesis using T4 DNA Polymerase" pages 211-217

## Description

### FIELD OF THE INVENTION

The present invention is related to a process for amplifying and detecting existing nucleic acid sequences if they are present in a test sample. More specifically, it is related to a process for producing any particular nucleic acid sequence from a given sequence of DNA or RNA in amounts which are large, when compared to the amount initially present. The DNA or RNA may be single or double-stranded, and may be a relatively pure species or a component of a mixture of nucleic acids. The process of the invention utilizes a repetitive reaction to accomplish the amplification of the desired nucleic acid sequence.

### BACKGROUND OF THE INVENTION

For diagnostic applications in particular, the target nucleic acid sequence may be only a minute portion of the total pool of DNA or RNA in a sample to be screened, so that it may be difficult to detect the presence of the target nucleic acid sequence using nonisotopically labeled or end-labeled oligonucleotide probes. Thus, diagnostic tests employing DNA probes to detect rare species of nucleic acids are often not sensitive enough to be practical for use outside of the research laboratory.

One attempt to overcome the sensitivity problem is the polymerase chain reaction (PCR) method, described in U.S. Patent Nos. 4,683,195 and 4,683,202 ("the '195 and '202 patents"). This method proceeds basically, as follows:
a) treating a sample suspected of containing the target nucleic acid sequence of interest with one oligonucleotide primer for each strand of the target nucleic acid sequence, under hybridizing conditions and in the presence of a polymerase, e.g., the Klenow fragments of Escherichia coli DNA polymerase-I, such that an extension product of each primer is synthesized if the target nucleic acid sequence is present;
b) placing the sample after step (a) under denaturing conditions to separate any primer extension products that were synthesized from the templates on which they were synthesized to produce single-stranded molecules;
c) treating the single-stranded molecules generated from step (b) with the primers of step (a) under the conditions of step (a), such that the new primer extension products are synthesized using both the original target sequences and the primer extension products produced in step (a) as templates, thus resulting in the amplification of the target nucleic acid sequence.
Steps (a)-(c) may be conducted sequentially or simultaneously. In addition, steps (b) and (c) may be repeated until the desired level of sequence amplification is obtained. As discussed in U.S. Patent Nos. 4,683,195 and 4,683,202, the product of step (c) may be detected using probes.

The PCR method has a disadvantage in that it fails to completely overcome the sensitivity problem. The PCR method uses all four nucleotide bases to extend the primer fragments. Therefore, extension products may be created from other, non-target nucleic acid templates that may be present in the sample such as nicked, double-stranded DNA. The use of the PCR method results in considerable background of amplified DNA other than the target sequence(s).

As will be discussed in detail later, the present invention uses at least two oligonucleotides for each strand of target nucleic acid sequence and uses fewer than all four bases, thus reducing the problem of nonspecific, background amplification for a number of reasons. For example, when labeled nucleotides are used, the gap will be filled with labeled nucleotides if the nucleic acid target sequence exists in the sample and the irrelevant sequences will not be copied or labeled.

The polymerase chain reaction method also requires heat stable enzymes for the process to be automated, while the process of the present invention can be performed using heat-labile enzymes or without any enzymes, depending upon the particular embodiment. In addition, the detection of amplified nucleic acids produced in the PCR method often requires the use of gels or a capturing system, which are laborious detection methods. In contrast, the detection of the amplified sequences in the present invention is relatively simple. For example, a Sephadex column can be used to separate the joined, oligonucleotide products formed when the target sequence is present apart from the individual nucleotides. European Patent Application 0 246 864 discloses a method for detecting a target nucleic acid, said method involving hybridization of two nucleic acid probe molecules to the nucleic acid target strand, which nucleic acid probe molecules are such, that upon hybridization they are adjacent in a manner that the gap existing between the probe molecules can be filled by target directed polymerization of the gap, for example using a polymerase and subsequent linkage, for example by DNA ligase.

Other methods, beside the PCR method, exist for producing nucleic acids in large amounts from initially small amounts. For example, there is the method of subcloning a nucleic acid in the appropriate host system, where the desired nucleic acid is inserted into an appropriate vector which is used to transform the host. When the host is cultured, the vector is replicated, and hence more copies of the desired nucleic acid are produced. For a brief description of subcloning nucleic acid fragments, see Maniatis, T., et al., Molecular Cloning; A Laboratory Manual, Cold Spring Harbor Laboratory, pp. 390-401 (1982). See also the techniques described in U.S. Patent Nos. 4,416,988 and 4,403,036.

Other methods for synthesizing nucleic acids include the organic synthesis of a nucleic acid from nucleotide derivatives such as the methods described in U.S. Patent No. 4,356,270. Another example of the method for synthesizing nucleic acid is provided in U.S. Patent No. 4,293,652, which is a hybrid of organic synthesis and molecular cloning. The discussion of these and other methods in the '195 and '202 patents, as well as the patents listed above, are incorporated herein by reference.

### SUMMARY OF THE INVENTION

The present invention relates to a process for amplifying one or more specific target nucleic acid sequences present in a sample.

In one embodiment of the present invention, the amplification is accomplished by using two or more oligonucleotide complement pairs wherein at least one strand of these oligonucleotide pairs has a nucleotide sequence complementary to at least a portion of the target sequence. The oligonucleotides are selected so there is a gap of at least one base when the complementary strands of the "oligonucleotide complement pairs" and the target nucleotide are hybridized to one another. The gap between the oligonucleotide complement sequences is filled by a mixture of polymerase and ligase producing an oligonucleotide repair product. The resulting mixture of hybridized molecules is then placed under denaturing conditions.

After the strands separate during denaturation, the ligated oligonucleotide product can hybridize to its complementary strands from other oligonucleotide complement pairs, and then the gap is filled again. The process is repeated as often as is necessary to produce the desired amount of oligonucleotide repair product. In one embodiment of the present invention, the enzymes are immobilized on a polymeric support.

The present method is especially useful, for amplifying sequences indicative of a genetic disorder and rare species of nucleic acid present in a mixture of nucleic acids, and further permits the effective detection of such nucleic acid sequences. The present invention provides a process for amplifying at least one specific nucleic acid sequence in a sample of a nucleic acid or a mixture of nucleic acids. Each nucleic acid target could consist of one strand (RNA) or two separate complementary strands (DNA) of equal or unequal length.

The process is accomplished as follows:
a) Treating the sample with oligonucleotide complement pairs A,A' and B,B' under hybridizing and gap filling conditions. A is an oligonucleotide and A' is an oligonucleotide that is complementary to A. B is an oligonucleotide and B' is an oligonucleotide that is complementary to B. When the set of two pairs A,A' and B,B' are hybridized to nucleic acid target, A and B, which hybridize to one strand of the target forms a gap of one or more bases between them. Also, A' and B' form a gap of one or more bases between them. The gap is filled with labeled or unlabeled base(s) such that A and B become one strand with a continuous base sequence with one or more extra labeled or unlabeled bases, (A-Q-B), where Q is(are) the base(s) that fill the gap. Q could also be a base modified to be resistant to degradation caused by the 3'-->5'exonuclease activity of polymerases used to fill the gap. Also, A' and B' are now one strand with a continuous base sequence with one or more extra labeled or unlabeled bases, (A'-Q'-B'), where Q' is(are) the base(s) that fill the gap. Similarly, Q' could also be a modified bases resistant to degradation caused by the 3', 5' exonuclease activity of polymerases. For any continuous base sequence A-Q-B or A'-Q'-B', Q or Q' can be composed of only one set of base pairs for any specific sequence, i.e., A-T, A-U, G-C, or derivatives of these bases. A-Q-B and A'-Q'-B' and are now joined, oligonucleotide products and can now serve as "target" sequences for other oligonucleotide complement pairs. When the joined, oligonucleotide product is formed by this gap-filling, ligated process it is termed an "oligonucleotide repair product."
b) Treating the sample under denaturing conditions to separate the oligonucleotide repair products from their targets, if the nucleic acid target sequence(s) is(are) present.
c) Treating the sample as in step (a) with oligonucleotide complement pairs A,A' and B,B' under hybridizing and gap-filling conditions such that an oligonucleotide repair product is obtained using each of the single strands produced in step (b), resulting in the amplification of the specific nucleic acid target sequence(s) if present.

The steps may be conducted sequentially or simultaneously. In addition, steps (b) and (c) may be repeated until the desired level of sequence amplification is obtained.

This invention is also related to methods for the detection of the amplified specific nucleic acid sequence and diagnostic kits applicable thereto.

### BRIEF DESCRIPTION OF THE DRAWINGS

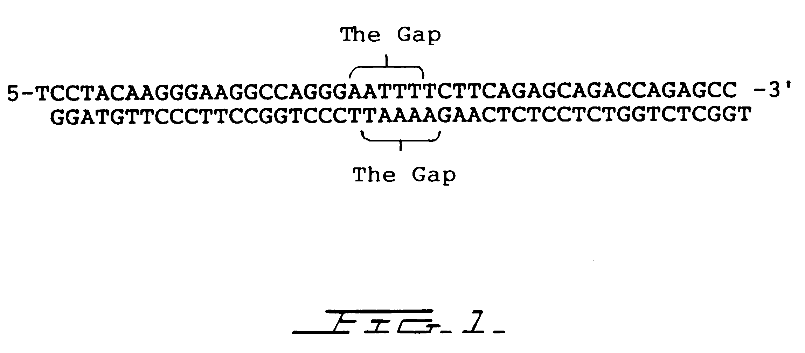

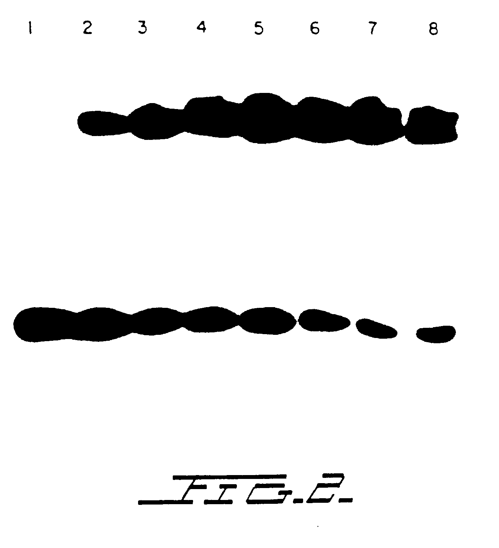

Fig. 1 illustrates a 48 base pair length sequence of HIV from GAG region desired to be amplified. The base pairs which fill the gap is depicted above and below the 48 base pair.

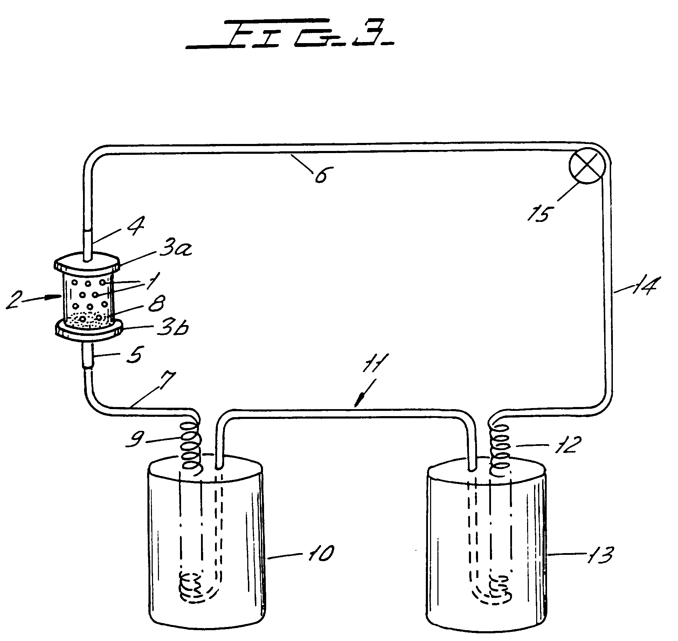

Figure 2 shows a 12% polyacrylamide-7M urea gel, where each lane consists of a cycle that includes: boiling the mixture of human immunodeficiency virus (HIV) plasmid target with labeled oligonucleotides, attached to photoreactive compounds, quick cooling the mixture, to 37°C and irradiating the mixture for five minutes.

Fig. 3 depicts an apparatus for carrying out one embodiment of the method of the present invention wherein the enzymes used are immobilized on a polymeric support.

### DETAILED DESCRIPTION OF THE PREFERRED EMBODIMENTS

### Definitions

The term "oligonucleotide" as used herein, is defined as a molecule comprised of three or more deoxyribonucleotides or ribonucleotides, preferably more than five.

The term "blunt end" is defined as two oligonucleotides that have sequences complementary to each other and at least one end of equal length when hybridized together.

"Sticky end" as defined in this application refers to two oligonucleotides that have sequences complementary to each other and at least one end of unequal length when hybridized together. The categories of blunt end and sticky end are illustrated below.

The term "two oligonucleotide complement pairs" are at least four different oligonucleotides which are designated, for example, A,A' and B,B' wherein oligonucleotide A has a base sequence complementary to A', and oligonucleotide B has a base sequence complementary to B'. Each pair could be equal or unequal in length, which is illustrated as follows:
- Category 1.: Oligonucleotide Complement Pairs with Blunt Ends 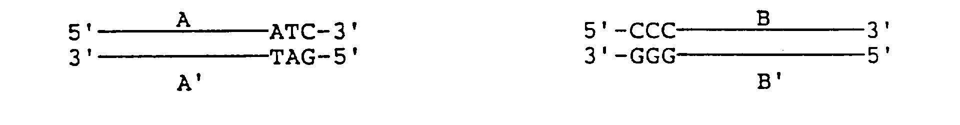
A                                        B
5'ATC-3'                        5'-CCC3'
3'TAG-5'                        3'-GGG5'
A'                                       B'
- Category 2.: Oligonucleotide Complement Pairs Each Having One Blunt and One Sticky End 
E
5'———————ATCA-3'
3'———————TAG-5'
E'

F
5'-CCC———————3'
3'-AGGG———————5'
F'
Two oligonucleotide complement pairs with a sticky end on one end of each pair are preferred as the bases for forming the collection of amplified oligonucleotides of the present invention. It would be understood that more than two oligonucleotide pairs could be used in the process of the present invention.

The term "gap" as used herein refers to the absence of one or more bases between A and B, E and F, or between A' and B', E' and F'. If more than two pairs are used then there may be more than one gap. The gap is created when the pairs are hybridized to the nucleic acid target, as for example: 
Line 1:   5'——————ATCATATCCC——————3'
Line 2:   3'——————TAG    GGG——————5'
                  (the gap)
 Line 1 is the sequence of the nucleic acid target, and line 2 represents oligonucleotide complements A' and B', as hybridized to the nucleic acid target sequence. If the target sequence is double-stranded, oligonucleotides A and B will form a hybrid with the sequence complementary to the target sequence shown above in line 1.

### A. GAP-FILLING, LIGATION AMPLIFICATION

In one embodiment of the process for amplifying nucleic acid sequences of the present invention, at least two oligonucleotide complement pairs are combined with the sample suspected or known to contain the target nucleic acid sequence of interest under hybridizing conditions. The oligonucleotide complement pairs are selected so that there is a gap in the nucleotide sequence of at least one base between the two complements when the two complements are hybridized with the nucleic acid target sequence. The gap is then filled and the two oligonucleotides are ligated together, producing an oligonucleotide repair product. The process of gap-filling plus ligation is analogous to the repair of mismatched bases, and other errors that occur during DNA replication, repair of UV damage, and other processes in vivo. The nucleic acid target sequence and the oligonucleotide repair product sequence may then be separated and the process repeated over and over again until the desired level of amplification has been achieved. To avoid a problem of background synthesis occurring during the gap filling step, the two or more oligonucleotide complement pairs are selected so that the gaps between them will require less than all four bases to fill in the gap, preferably one set of complementary bases, namely A-T, A-U, or G-C. Without all four bases, random synthesis would not be initiated by nucleic acid sequences that may have nicks or are in the process of replication or transcription.

### 1. Nucleic Acid Target Sequences

The process of the present invention can produce exponential quantities at least one specific nucleic acid sequence relative to the number of reaction steps involved, provided that (a) at least part of the nucleic acid target sequence is known in sufficient detail that oligonucleotide pairs can be synthesized which can hybridize to it, or (b) the target sequence can be isolated in large enough quantities to produce enough oligonucleotide complement pairs for use in the process. Any source of nucleic acid can be utilized as the source of the target nucleic acid sequence, in purified or nonpurified form. For example, the process may employ either single-stranded or double-stranded DNA or RNA. In addition, a DNA-RNA hybrid which contains one strand of each may be utilized. A mixture of any these nucleic acids may be employed. The specific nucleic acid sequence to be amplified may be only a fraction of a larger molecule. It may be a minor fraction of a complex mixture, such as a portion of the HIV (human immunodeficiency virus) gene integrated in the genomic DNA of an infected person, or bacterial nucleic acid present in very low quantities in a particular biological sample.

To determine the sequence of the target sequence(s), or as a sample to be tested, the nucleic acid or acids of interest may be obtained from any source, for example, DNA or RNA, isolated from bacteria, viruses, yeast and higher organisms such as plants or animals, from plasmids such as PBR 322 and M13, from cloned DNA or RNA by a variety of techniques known to those skilled in the art. DNA may also be extracted from cells grown in tissue culture by techniques such as those described by Maniatis et al., Molecular Cloning, a Laboratory Manual (New York: Cold Spring Harbor Laboratory, 1982), pp.280-281.

### 2. Oligonucleotide Complement Pairs

The oligonucleotide complement pairs are preferably oligodeoxyribonucleotides. In addition, the pairs must be long enough to hybridize to the nucleic acid target sequence(s). The length of the complement pairs can vary from four bases to hundreds of bases. A short oligonucleotide generally requires cooler temperatures to form stable, hybrid complexes. The oligonucleotide sequences synthesized are selected so that two oligonucleotide complement pairs would both hybridize to the target sequence and yet leave a gap of one or more bases. If the target sequence is single-stranded, only one half of each oligonucleotide pair would hybridize to the target. More than two pairs of oligonucleotide complements can be employed in the process of the invention as long as the amplification will remain specific for the nucleic acid target sequence(s).

The oligonucleotide complement pairs may be prepared using any suitable method, e.g., phosphoramidites (Applied Biosystems Inc.) may be used as starting materials and may be synthesized as described by Beaucage, et al., Tetrahedron Letters, 22, 1859-62 (1981) and phosphorylated at 5'-end by methods well-known in the art.

### 3. Denaturation

The strand separation can be accomplished by any suitable denaturing method including physical, chemical or enzymatic means. One physical method of separating the strands of the nucleic acid involves heating the nucleic acid until it is completely (more than 99%) denatured. Typical heat denaturation may involve temperatures ranging from about 80°C to 105°C, for times ranging from about 1 to 10 minutes. Strand separation may also be induced by an enzyme from the class of enzymes known as helicases or the enzyme RecA, which has helicase activity and in the presence of riboATP is known to denature DNA. The reaction conditions suitable for separating the strands of nucleic acids with helicases are described by Cold Spring Harbor Symposia on Quantitative Biology, Vol. XLIII "DNA Replication and Recombination" (New York: Cold Spring Harbor Laboratory, 1978), B. Kuhn et al., "DNA Helicases", pp. 63-67, and techniques for using RecA are reviewed in C. Radding, Ann. Rev. Genetics, 16:405-37 (1982).

### 4. Gap-Filling/Ligation Steps

Generally, the gap-filling and ligation steps occur in a buffered aqueous solution, preferably at a pH of 7-9, most preferably at a pH 7.5. The oligonucleotide complement pairs will be present in molar excess of about 10⁵-10¹⁵ pairs per nucleic acid target sequence. The exact amount of the pairs to be used in diagnostic purposes may not be known due to uncertainty as to the amount of the nucleic acid target in a sample. However, using an average amount of 10¹⁵ oligonucleotide complement pairs is applicable in a typical diagnosis assay format. A large molar excess is preferred in any case to improve the efficiency of the process of the invention.

In the process of the invention, typically only two complementary deoxyribonucleoside triphosphates would be used in the gap-filling step, dATP and TTP, or alternatively, dCTP and dGTP. Sticky-ended oligonucleotide complement pairs can be selected, so that only one type of deoxyribonucleoside triphosphate is required to fill in any gaps. Sticky-ended complement pairs can also be selected so that two complementary nucleotides and one other nucleotide are used to fill the gap.

It is preferred to utilize modified nucleoside triphosphates known in the art to be resistant to the exonuclease activity of polymerases, as described by D. Shortle et al., Proc. Natl. Acad. Sci. USA, 79: 1588-92 (1982); T. A. Kunkel, ibid., 78: 6734-38, (1981); F.R. Bryant et al., Biochemistry 18: 2825-28 (1979). Such molecules could be thymidine 5'-O-(1-thiotriphosphate) (TTP [αS]), 2'-deoxyadenosine 5'-O-(1-thiotriphosphate) (dATP [αS]) and the thioderivatives of deoxycytidine, deoxyguanidine, deoxyuridine and of cytidine, guanidine, adenosine, thymidine, and uracil. Other derivatives of these bases that are resistant to nuclease activity are suitable, such as α-imido derivative of triphosphate bases.

Sufficient deoxyribonucleotide triphosphates are added to the gap-filling/ligating mixture in adequate amounts and the resulting solution is heated to about 90°C to 100°C for approximately one to five minutes, preferably from one to three minutes. The solution is then allowed to cool to room temperature to allow hybridization to occur. To the cooled solution, appropriate catalysts are added to induce the filling and sealing of the gap under conditions known in the art. For example, known DNA polymerases can be used for the gap-filling and known DNA ligases can join the resulting product after the polymerase has filled the gap. The gap-filling ligation process may occur at from 4°C up to a temperature at which the catalytic agents no longer function efficiently; the temperature is generally no greater than about 40°C. Thus, for example, if T4-DNA polymerase and T4-DNA ligase are used, the temperature is generally no greater than about 40°C (most conveniently, the reaction occurs at room temperature or even at 4°C). If heat insensitive enzymes are used, then the process could occur at the melting temperature of the hybrids templates.

The catalytic agent may be any compound or system which will function to fill the gap between the two or more oligonucleotides hybridized to the nucleic acid target. Enzymes suitable for this purpose include E. coli DNA polymerase-I, Klenow fragment of E. coli DNA polymerase-I, T4-DNA polymerase, reverse transcriptase for adding bases to fill in the gap, T4-DNA ligase to join the oligonucleotide(s), plus nucleotides added by the polymerase, to the other oligonucleotide complements, forming a joined oligonucleotide product, which in this embodiment is termed the oligonucleotide repair product.

The oligonucleotide repair products hybridized to the nucleic acid targets are in a double-stranded form. In the next step, the strands of the double-stranded molecule are again separated as described above to provide single-stranded molecules.

In order to generate a mode of amplification, the joined oligonucleotide molecules can hybridize to two more oligonucleotide complement pairs. If necessary, additional enzymes, appropriate deoxyribonucleotide triphosphates, and oligonucleotides may be added for the reaction to proceed.

The steps of strand separation, gap-filling and ligation can be repeated as often as needed to produce the desired amount of the specific joined, oligonucleotide product assuming that one copy of single-stranded nucleic acid target sequence was present in the mixture at the beginning. As long as these steps will be repeated, the amplification of the specific nucleic acid target sequence will take place in an exponential way. This process could be used to amplify other nucleic acid target sequences, by adding different oligonucleotide complement pairs (or triplets, etc.) that hybridize to different specific nucleic acid target sequences without changing other conditions involved.

This particular embodiment of the invention could be performed in a stepwise fashion, in which new reagents are added after each step, or simultaneously, in which all reagents are added at once. The reagents can also be added after a given stage. Each step of the embodiment using heat-stable enzymes will occur sequentially regardless of the initial concentration of all the reagents. When heat-sensitive enzymes are used, it is necessary to add the gap-filling and sealing agents after every strand separation (denaturation) step.

In one embodiment of the present invention, the catalytic agents are immobilized on polymeric supports. After the gap-filling and ligation step, the products can be eluted from the immobilized enzymes and denatured to single strands. At this point, the nucleic acid target sequence(s) and joined, oligonucleotide product(s) are available to form hybrids with additional, oligonucleotide complement pairs. These new hybrids are then transferred back to the immobilized enzymes to form joined products. The steps of the reaction may be carried out stepwise or simultaneously and may be repeated as often as desired.

For smaller oligonucleotide complements, for example, 6-10 bases, heat-sensitive enzymes could be employed in a simultaneous procedure, since the melting temperature of double-stranded oligonucleotides of this range is around 40°C, and heat-sensitive enzymes are active at this temperature.

If heat-stable gap-filling and sealing agents are used, such as thermostable polymerase and ligase, then the process could be employed at an elevated temperature, preferably 60-90°C depending on the heat-stable enzymes. The temperature would also be determined by the temperature at which there will be an equilibrium between single and double-stranded nucleic acids. Such a heat-stable polymerase is described by A.D. Kaledin, et al., Biokhimiya, 45, 644-51 (1980). A heat-stable polymerase and ligase could be extracted from Thermus thermophilus.

After the appropriate period of performing the process has passed, and the desired amount of the oligonucleotide repair product has accumulated, the reaction may be stopped by inactivating the enzymes in any known manner or separating the components of the reaction on spun or Sephadex columns, by filtration, or by gel electrophoresis as known in the art.

The process of this invention may be employed as an automated process. To do this, the reaction is cycled through a denaturing step, a reagent addition step, and a reaction step. Also the process may be conducted continuously by adding the reagents automatically by a pump after the denaturing step, while the heating-cooling step could be performed by a specially designed, controlled heater block.

The present invention is demonstrated diagrammatically below. Using two oligonucleotide complement pairs that have one sticky end each (Category 2.) 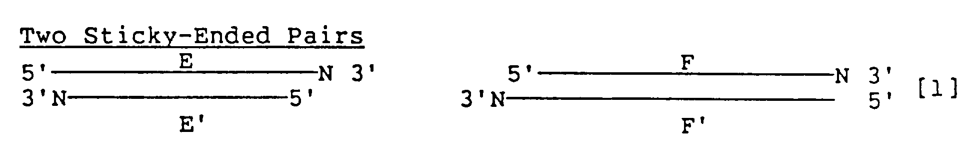
Two Sticky-Ended Pairs
E F
5'N 3' 5'N 3'
3'N5' 3'N5' [1]
E' F'
 N is a nucleotide modified to protect the strands from degradation by 3' exonucleases, such as that found as part of T4-DNA polymerase. Oligonucleotide complement pairs E, E' and F, F' are selected or synthesized to correspond to sections of the complete nucleic acid target sequence R. R is a double-stranded DNA comprising complementary strand R+ and R- represented as: 
5'····——————————CATATC——————————····3' R+
[2]
3'····——————————GTATAG——————————····5' R-
 When [R] is mixed with a molar excess oligonucleotides of complement pairs E, E' and F, F' under conditions where the double-stranded molecules are denatured and then are permitted to rehybridize, R+ forms stable, double-stranded hybrids with E' and F', while R- forms hybrids with E and F as illustrated by: 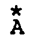 
5'····——————CATATC——————····3' R+
3' N——————G  AG——————5'
E'  *  F'
[3]
*
5'——————CA  C——————N 3'
E  F
3'····——————GTATAG——————····5' R-
 represents the modified nucleotide N of diagram [1] such as [NTP's αS].

Next, TTP, dATP, E. coli. Klenow fragment and T4-DNA ligase are added to mixture, permitting the gap in the double strands to be filled and sealed, producing: 
5'····——————CATATC——————····3' R+
3'N——————GTATAG——————5'
E' F'
joined,
nucleotide
product
[4]
E * F
5'——————CATATC——————3'
3'····——————GTATAG——————····5' R-
joined,
nucleotide
product
 In this particular example, the Klenow fragment adds the nucleotides to the 3' of F' and the ligase joins the added nucleotides to the 5' end of E'. E and F are joined in the same fashion.

In the first cycle, two new single-stranded DNA have been produced. After the next denaturating step, the molar excess of the two oligonucleotide complement pairs can hybridize to the original nucleic acid targets as well as to the oligonucleotide repair product formed in the first cycle, thus four more oligonucleotide repair products are formed in the second cycle. The formation of the new joined oligonucleotides product will proceed exponentially.

In this process, dATP and TTP could be labeled with radioactive labels, such as ³P, ³⁵S, or ¹⁵I. Non-radioactive labels can also be used. For example, TTP could be replaced by Biotin-dUTP (Enzo Biochem), and dATP could be replaced by Biotin-dATP as described by G. Gebeyehu et al., Nucleic Acid Res. 15, 4513-34 (1987).

In the case when blunt-ended oligonucleotide pairs in solution are used (category 1), blunt-end ligation could occur between the set of two pairs in solution. However, since only the bases which filled the gap are labeled, the blunt end ligation products will not be counted in the detection step.

### B. DETECTION OF THE JOINED, OLIGONUCLEOTIDE PRODUCT

The joined, oligonucleotide product can be detected by any number of methods for detecting labeled molecules and/or molecules having a particular length or sequence. For instance, the reaction mix may be passed through a Sephadex column to separate the labeled NTPs and the joined oligonucleotide products. Since individual nucleotides are much smaller than the length of the two or more oligonucleotide complement pairs, the detection of amplified material should be fairly simple based on size alone.

Another technique to detect amplified sequence would require the construction or isolation of probes that share complementary sequences with enough of each and every oligonucleotide complement pair to bind and hold the joined product preferentially. Such probes can be immobilized on any suitable substrate. They may also be labeled differentially if desired.

The present invention may be used for in vitro diagnostics. The process of amplifying nucleic acid sequences enables the detection of specific nucleic acid sequences associated with infectious disease, genetic disorders or cellular disorders such as cancer. Amplification is particularly useful when the amount of nucleic acid target available for diagnosis is in minute quantities, as, for example, in the prenatal diagnosis of sickle cell anemia, which requires obtaining DNA from fetal cells. Furthermore, it is within the ability of those skilled in the art, that the length and sequences of the oligonucleotide complements can be varied to detect deletions and/or mutations in genomic DNA from any organisms. These small changes are important in the diagnosis of such conditions as cystic fibrosis, α-thalassemia, β-thalassemia and the like.

The process of the invention may also indicate the presence of various agent like pathogenic viruses including human immunodeficiency virus (HIV), herpes, hepatitis B and bacteria including Salmonella and Chlamydia.

U.S. Pat No. 4,358,535, issued to Falkow, describes the use of specific DNA hybridization probes for the diagnosis of infectious diseases. A problem inherent in the Falkow procedure is that a relatively small number of pathogenic organisms may be present in a clinical sample from an infected patient and the DNA extracted from the pathogens may constitute only a very small fraction of the total DNA in the sample. Specific amplification of suspected sequences in a sample prior to the hybridization to the probes and the immobilization on filters samples could greatly improve the sensitivity and specificity of these procedures.

In a further embodiment, the amplified species could be detected by a simple method as follows: A capture oligonucleotide probe that has a nucleic acid sequence which is complementary to both E and F or E' and F' or to each one of them separately could be used to capture the amplified labeled products.

In another embodiment, the deoxyribonucleotide triphosphates involved in the process could be radioactively labeled such as with ³P, ³⁵S, ¹⁵I and others with non-radioactive labeling.

Another means to facilitate the clinical use of DNA probes for the diagnosis of infectious diseases is the substitution of non-radioactive labels for radioactive ones. As described in EP 63,879 to Ward, biotin-containing DNA probes are detected by chromogenic enzymes linked to avidin or biotin-specific antibodies. This type of detection is convenient, but relatively insensitive. The combination of DNA amplification by the present method and the use of Bio-dUTP to label the bases that filled the gap could provide the convenience and sensitivity required to prepare useful diagnostic kits and to overcome the difficulties associated with both Falkow and Ward procedures when these techniques are applied in a routine clinical setting.

The use of the Falkow and Ward methods, the synthesis of oligonucleotides, the calculation of the number of sequences amplified per cycle, and other matters that pertain generally to amplification of nucleic acid sequences are described in the '195 and '202 applications and these applications are incorporated herein by reference.

The invention will now be illustrated by examples. The examples are not intended to limit the scope of the present invention. In conjunction with the general and detailed description above, the examples provide further understanding of the present invention and outlines some aspects of the preferred embodiments of the invention.

### EXAMPLE 1

The desired sequence to be amplified using the gap-filling/ligating embodiment of joining the oligonucleotides is a 48 base pair sequence that coded for HIV at GAG region 2106-2153. This sequence is chosen for its G-C content which is about 52% and has the following sequence: 
5'-TCCTACAAGGGAAGGCCAGGGAATTTTCTTCAGAGCAGACCAGAGCC-3'
  3'-GGATGTTCCCTTCCGGTCCCTTAAAAGAAGTCTCGTCTGGTCTCGGT-5'
 The following four oligodeoxyribonucleotides are examples of the many blunted-ended sequences that can be used for amplification according to the method of the invention.

A - 5'-TCCTACAAGGGAAGGCCAGGG-3'
A'-   3'-GGATGTTCCCTTCCGGTCCCT-5'
B - 5'-TCTTCAGAGCAGACCAGAGCC-3'
B'-   3'-GAAGTCTCGTCTGGTCTCGGT-5'

The blunt ends are created after incorporation of 2'-deoxyadenosine 5'-0-(1-thiotriphosphate) (dATP[αS]) at the 3' end of each sequence. A gap of 5 base pair exists between the oligonucleotides A and B or A' and B' when these strands are hybridized to their complementary strand of the target sequence shown above.

Similarly, the following four oligodeoxyribonucleotides are suitable sticky-ended sequences that may be utilized in the process of the invention.

E - 5'-TCCTACAAGGGAAGGCCAGGGA-3' 22 bases
E'- 3'-GGATGTTCCCTTCCGGTCCCTTA-5' 22 bases
F - 5'-TCTTCAGAGCAGACCAGAGCC-3' 21 bases
F'- 3'-GAAGTCTCGTCTGGTCTCGGT-5' 21 bases

All of oligodeoxribonucleotides described above are synthesized and purified by the following procedure.

### I. Automated Synthesis Procedures.

The 2-cyanoethyl phosphoramidites are purchased from Applied Biosystems Inc. The procedure includes condensation of nucleoside phosphoramidites to a nucleoside-derivatized controlled Pore glass bead support (500Å) 30 mg, using DNA synthesizer from Applied Biosystems Inc., Type 380B-02. The cycles includes detritylation with 2% trichloroacetic acid in dichloromethane; condensation using tetrazol as an activating proton donor; capping with acetic anhydride and dimethylaminopyridine; oxidation of the phosphite to the phosphate with 0.1 M I₂/H₂O/Lutidine/Tetrahydrofuran, following detritylation using 2% trichloroacetic acid in dichloromethane. Cycle time is approximately 30 minutes. Yields at each step are essentially quantitative and are determined by collection and spectroscopic examination of the dimethoxytrityl alcohol released during detritylation.

### II. Oligodeoxyribonucleotide Deprotection And Purification Procedures

The solid support is removed from the column and exposed to 1 ml concentrated ammonium hydroxide at 60°C for 16 hours in a closed tube. Ammonia is removed and the residue is applied to a preparative 12% polyacrylamide gel using a Tris-borate buffer (pH 8) containing 7M urea. Electrophoresis is carried out at 20 volts/cm for 5 hours after which the band containing the product is identified by UV shadowing of a fluorescent plate. The band is excised and eluted with 1 ml double distilled water overnight at room temperature. This solution is filtered and the supernatant is extracted (3 x 300 microliter) with n-butanol. The water phase is placed on a Sephadex G50 column (Pharmacia) (1x 10cm). The elution is monitored by UV absorbance at 260 nm and the appropriate fraction collected, quantitated by UV absorbance in a fixed volume and evaporated to dryness at room temperature in a vacuum centrifuge.

### III. 5'- Phosphorylation of A',B, E' and F sequences

In order to phosphorylate 5'-ends of A',B, E' and F, a new-phosphorylating reagent as described by T. Horn et al., Tetrahedron Letters 27, 4705-08 (1986) is used. Only the ends of the nucleotides that are to be joined together will be phosphorylated.

The synthesis of A',B, E' and F is described as in I. Automated Synthesis Procedure, but after the last cycle, the phosphorylating reagent 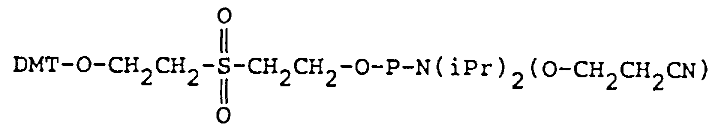 (Glen Corp. Inc.) (DMT is dimethoxytrityl group; iPr is isopropyl) is condensed to each of the sequences A',B, E' and F using tetrazol as an activating proton donor, followed by capping with acetic anhydride and dimethylaminopyridine; oxidation of the phosphite to the phosphate with 0,1M I₂/H₂O/Lutidine/Tetrahydrofuran, following detritylation using 2% trichloroacetic acid in dichloromethane. Cycle time is approximately 30 minutes.

The 5'-phosphorylated A',B,E' and F are purified and quantitated by the procedure described in II above.

### EXAMPLE 2

This experiment illustrates the amplification of the 48 base pair sequence of Example 1 by using a set of two blunt-ended oligonucleotide complement pairs, where a gap of 5 bases (A,T) exists between the pairs. In the first step the polymerase will incorporate dATP[αS] in order to protect the complement pairs from the 3'-exonuclease activity of DNA polymerase I and to form blunt-ends.

Amplification of the 48 base pairs starts from step 2.

### Step 1:

50 microliters containing 1mM MgCl₂, 20mM 2-mercaptoethanol, 100 mg of bovine serum albumin per ml, 0,1mM ATP, and 0,1mM of dATP[αS]) (Sigma) are added to a 60 microliter buffer solution containing 50mM Tris-HCl, pH 7.5.

To this solution are added 5 microliters of solution containing 100 picomoles each of A and B' and phosphorylated A' and B and 0.1 picomole of the 48 base pairs to be amplified. The resulting solution is heated to 100°C for 2 minutes, which allows separation of the strands and hybridization of the A, A', B, and B' to the target. Then, 4 units of Klenow fragment of E. coli DNA polymerase-I are added and the reaction is incubated for 10 minutes at room temperature.

The two pairs are now blunt-ended and protected against exonuclease activity of polymerase.

### Step 2:

In order to produce and amplify the joined, oligonucleotide products, the following sub-steps are employed.
a. Add 1 nanomole each of dATP and TTP and 100 picomoles of each of α-³P-dATP and α-³P-TTP (Specific activity 1000 cpm/picomole).
b. Heat for 2 minutes to 100°C.
c. Cool to room temperature for 2 minutes.
d. Add a mixture of 4 units of Klenow fragment of E. coli DNA polymerase-I and 2 units of T4-DNA ligase.
e. Incubate for 10 minutes at room temperature.

Sub-step (a) in the cycle can be omitted if a sufficient amount of nucleotide triphosphates are present initially. The cycle is repeated 22 times. 2 microliter aliquots from cycles 2, 4, 7, 10, 13, 16, 19, and 22 are applied to a 12% Polyacrylamide gel, 7M urea using 0.089M Tris-borate buffer, pH 8.3. Electrophoresis is carried out at 20 volts/cm for 5 hours. The gel is exposed to sensitive film (Kodak) for 5 hours.

The resulting reaction mixture is loaded on a Sephadex G40/50 column (1X10cm) (Pharmacia) and eluted with double distilled water. Two distinct peaks are separated and monitored by a Geiger counter. The first peak, appearing after elution of 5-7 ml of water, consists of the amplified 48 base pairs, and the second peak, appearing after elution of 14-18 ml of water, consists of α-³P-dATP and α-³P-TTP.

In this example, steps 1 and 2 are employed separately. However, the two steps can be combined into one step, so that the cycling could start immediately by using a mixture of dATP[αS] that could be added in Step 2, sub-step (a) of the cycle, or, alternatively, the protected blunt-ended pairs could be prepared separately, purified, quantitated, and used as desired.

### EXAMPLE 3

This example illustrates a non-radioactive labeling during the amplification protocol of this invention. A 100 microliters solution containing 1mM MgCl₂, 20mM 2-mercaptoethanol, 100mg of bovine serum albumin per ml and 50mM Tris-HCl(pH 7.5) is prepared. 5 microliter of a solution containing one nanomole of each of A, A', B, and B' where B and A'are phosphorylated at their 5'-ends, and 10 picomole of 48 base pair as prepared in Example 1 were added. 1 nanomole of each of dATP and Bio-11-dUTP (Enzo Biochem) and 100 picomoles of dATP [αS] are also added. The number 11 designated a linker arm of 11 atoms.

The resulting solution is subjected to cycles as described above e.g., the mixture is heated to 100°C for 3 minutes and allowed to cool to room temperature for 2 minutes, whereupon 4 units of Klenow-fragment of E. coli DNA polymerase-I and 2 units of T4-DNA ligase are added. The reaction mixture is incubated for 5 minutes at room temperature. The cycle is repeated 20 times as described in the previous experiment.

The reaction mixture is then diluted with 100 microliter of 2M ammonium acetate buffer, pH 7.5. The mixture is serially diluted 4-fold using 1M ammonium acetate as a diluent. Aliquots (50 microliter) of the amplified DNA solution is added to the wells of 96-well Dynatech Immulon II microtiter plates (Dynatech). The plates are sealed and incubated for 90 minutes at 37°C to bind the amplified DNA to the plates. After the incubation, the wells are washed at room temperature twice with 200 microliters aliquots of 0.3M NaCl and 0.03M sodium citrate, pH 7.0, and once with 200 microliter of the same buffer containing 0.1% Triton X-100. The wells are treated for 30 minutes at room temperature with aliquots (200 microliter) of a solution containing 10 mM sodium phosphate (pH 7.4), 0.5 M NaCl, 2% BSA, 0.1% Triton X-100 and 5mM EDTA (blocking solution).

After removing of the blocking solution from the wells, aliquots (50 microliter) of a solution containing horseradish peroxidase-labelled avidin (Vector Labs) is added to each well. The peroxidase-labelled avidin is diluted in PBS, 0.1% Triton X-100 according to the manufacturer's instructions.

The plates are incubated for 30 minutes at room temperature, then washed (4 x 200 microliter) with a solution of 10mM sodium phosphate (pH 7.4), 0.5 M sodium chloride 0.1% Triton X-100 and 5mM EDTA and then (1 x 200 microliter) with PBS containing 1mM EDTA.

The DNA-biotinylated probe-labelled avidin complexes are detected by adding to each well 150 microliter of a substrate reaction mixture containing 1.6 mg/ml o-phenylenediamine (Sigma) and 0.0125% hydrogen peroxide in 0.1 M sodium phosphate buffer adjusted to pH 6.0 by 0.05M citric acid solution. The plates are incubated for 30 minutes at room temperature in the dark and then the reaction is stopped by the addition of 4N sulfuric acid (50 microliter).

The contents of the plates were read in an Intermed Immuno Reader NJ-2000 spectrophotometric plate reader at wavelength 490 nm. The results are shown in Table 1:

**Table 1**

| Absorbance (O.D. 490) | | |
|---|---|---|
| [Amplified DNA] | | Control^{(a)} |
| 1 nmole | over | 0.019 |
| 0.5 nmole | over | 0.025 |
| 0.250 nmole | over | 0.016 |
| 0.125 nmole | over | 0.012 |

| | | |
|---|---|---|
| (a) Control means 100 picomoles of Bio-dUTP were fixed on the wells using ammonium acetate. | | |

### EXAMPLE 4

This example illustrates amplification using sticky-ended oligonucleotide complement pairs (E, E', F and F' as described). The desired sequence to be amplified is the same 48 base pair sequence as prepared in Example 1. In this experiment the incorporation of dATP[αS] the gap-filling and the ligation step all take place at once.

1mM MgCl₂, 20mM 2-mercaptoethanol, 100mg of bovine serum albumin per ml, 1.0 micromolar each of dATP[αS], TTP[αS], dATP and dTTP, and 100 picomoles of each of α-³P-dATP and α³P-TTP (Specific activity 1000 cpm/picomole) are added to a 100 microliter buffer solution containing 50 mM Tris-HCl, pH 7.5.

To this solution are added a 5.0 microliter solution containing 100 picomoles of each of E and F' and each of phosphorylated F and E' and 0.1 picomole of 48 base pairs to be amplified. The resulting solution is heated to 100°C for 3 minutes and allowed to cool to room temperature for 2 minutes, which allows the separation of the strands and hybridization of the set of two pairs to the target. Then 4 units of Klenow fragment of E. coli DNA polymerase-I and 2 units of T4-DNA ligase are added and the reaction incubated for 10 minutes at room temperature.

In order to cycle the process, the following steps are employed:
1. Heat for 3 minutes to 100°C.
2. Cool to room temperature for 2 minutes.
3. Add a mixture of 4 units of Klenow fragment of E. coli DNA polymerase-I and 2 units of T4-DNA ligase.
4. Incubate for 10 minutes at room temperature. The cycle is repeated 22 times.
The resulting reaction mixture is loaded on a Sephadex G40/50 column (1X10cm) (Pharmacia) and eluted with double distilled water. Two distinct peaks are separated and monitored by a Geiger counter. The first peak appearing after elution of 5-7 ml of water, consists of the amplified 48 base pairs and the second peak appearing, after elution of 14-18 ml of water, consists of α-³P-dATP and α -³P-TTP.

### EXAMPLE 12

This example illustrates the coupling of T4-DNA ligase and Polymerase-I to polymeric supports.

### I. Synthesis of the "linker arm".

1. O-(di-p-methoxytrityl)hexaethylene glycol. Hexaethylene glycol (Aldrich), (28.23 g, 100 mmole) is dissolved in dry pyridine (200 ml) and a solution of dimethoxytrityl chroide (16.95 g, 50 mmole) in dry pyridine (100 ml) is added dropwise under argon. The mixture is stirred at room temperature for 4 hours and the solvent is removed under reduced pressure (2 mm Hg). The oily yellow residue is extracted with ethyl acetate (300 ml) and washed with water (200 ml) and brine (2x200 ml). The organic layer is dried over anhydrous sodium sulfate and filtered. The solvent is removed and the residue is redissolved in dichloromethane (30 ml) which contain some pyridine (0.5 ml). This mixture is further purified by column chromatography on silica gel (5.5x45 cm) and eluted with a mixture of dichloromethane-methanol-pyridine (95:4:1: vol/vol). The product (23.3 g, yellow oil) has a RF=0.42 in the above solvents. The yield is (80%).
NMR = 7.46-7.20 (m, 9H, aromatic Hs), 6.80 (d, J=9.0 Hz, 4H, aromatic Hs), 3.77 (s, 6H, methoxy Hs), 3.66, 3.63 (2 s, 22H, CH₂-CH₂) 3.56 (t, J=5 Hz, sH, CH₂-OH), 3.21 (t, J=6.0 Hz, 2H, CH₂-DMT), 2.49 (wide s, 1H, OH). Mass spectrum: m/e 584 (molecular ion), 553, 507, 477, 303 (DMT group).

### 2. O'-DMT-hexaethyleneglycol O-Methyl N,N-diisopropyl phosphoramidite.

O'-DMT-hexaethyleneglycol (1) (5.84 g, 10 mmole) is dissolved in dry dichloromethane (30 ml) under argon. Dry N,N-di-isopropylethyl amine (5.16 g, 40 mmole) is added followed by dropwise addition of N,N-di-isopropyl O-methyl chlorophosphoramidite (Applied Biosystems) (1.97 g, 10 mmole). The solution is stirred at room temperature for 30 minutes, diluted with ethyl acetate (300 ml), washed with a saturated solution of sodium bicarbonate (100 ml) and with brine (100 ml). The organic layer is dried over anhydrous sodium sulfate and the solvent is removed under reduced pressure (15 mm Hg). Further drying is achieved by coevaporation with toluene (2x100 ml) and by lyophylization in dry benzene.

The product (7.21 g, yellow oil) has RF-0.63 in dichloromethane-methanol (95:5). The yield is (95%). NMR = 7.47-7.23 (m, 9H, aromatic Hs), 6.83 (d, J=9.0 Hz, 4H, aromatic Hs), 3.77 (s, 6H, methoxy), 3.77-3.59 (wide s, 22H, O-CH₂CH₂-O), 3.55 (d, J-12 Hz, 3H, CH₃-O-P), 3.21 (t, J=6.0 Hz, 2H, CH₂-O-DMT), 1.62 (d, 2H, CH₃-CH), 1.24 (d, J=7Hz, 12H).
Mass spectrum 744 (molecular ion).

### II. Attachment of the linker-arm to controlled pore glass.

Long chain alkyl amine-controlled pore glass (CPG), (Pierce), (500 A), (1 g), is placed in a sinter glass (medium) (50 ml), and washed with dry acetonitrile (3x10 ml) under argon. To the dried gel, a solution of 1H-Tetrazole (Aldrich), (0.5 g) in dry acetonitrile (12 ml), is added, followed by addition of a solution of O'-DMT-hexaethyleneglycol O-Methyl N,N-diisopropyl phosphoramidite (2 g) in dry acetonitrile (13 ml), under argon. The reaction mixture is agitated for 10 minutes followed by washings with tetrahydrofuran (3x25 ml). To the resulting gel, a capping solution containing acetic anhydride:tetrahydrofuran (1:1 vol/vol) (10 ml), and a solution of N,N-dimethylamino pyridine (0.5 g) in tetrahydrofuran (10 ml) are added, and the mixture is shaken for 6 minutes, followed by washings with tetrahydrofuran (3x20 ml). To the resulting solution an oxidation solution containing I₂ (0.1 Molar) in tetrahydrofuran : water : 2,6-lutidine (1:0.4:1), (10 ml) is added, and the mixture is shaken for 5 minutes, followed by washings with acetonitrile (3x20 ml). The measurement of the loading of the linker-arm on the controlled pore glass is done by adding a solution (1 ml) of para-toluenesulfonic acid in acetonitrile (0.1 Molar), to a 10 milligram of the derivative support, followed by measuring the absorbance at 498 nm. The loading is 185 micromoles of the linker-arm per 1 gram of the support. The dinethoxytrityl group is removed from the support, by a solution of trichloroacetic acid in dichloromethane (3%). A new linker-arm could be condensed to the previous unprotected linker-arm in order to obtain a longer linker-arm, as desired.

### III. Activation of the CPG-linker-arm with tresyl chloride.

The dried support-linker-arm (1 g), is placed in a sinter glass as above, and washed with dry acetone (3x50 ml), followed by addition of dry acetone (5 ml) and 260 microliters of dry pyridine. Tresyl chloride (Fluka), (180 microliters) is added to the suspension dropwise for 1 minute, under shaking. After reaction for 15 minutes at 0°C, the gel is washed twice with (20 ml) of the following, 30:70, 50:50, and 70:30 of 5 mM HCL:acetone (vol/vol) and finally with water, 50:50 water:acetone and acetone, (20 ml each) dried, and stored in desiccator until required.

### IV. Coupling of T4-DNA Ligase to tresylated CPG-linker-arm.

40 units of T4-DNA ligase (BRL), are dissolved in 0,1M NaH₂PO₄, pH 7.5, containing 1 mM EDTA, 50 micromolar ATP, 10% glycerol to a final volume of 200 microliters and kept at 40°C. 100 milligrams of dry tresylated CPG-linker-arm is transferred to the enzyme solution. The resulting gel slurry is mixed "end over end" at 4C for 16 hours. Residual reactive tresyl groups on the linker-arm are removed by adding 0,1M dithiotreitol, (2 hours, 4°C). After coupling, the gel is washed free of uncoupled enzyme by washing four times in 0.1 M NaH₂PO₄, pH 7.5, 1 mM EDTA, 10 mM dithiothreitol (DTT), 0.5 M NaCl followed by two washes in 0.1 M NaAc, pH 5.0. Finally, the gel is equilibrated in 66 mM Tris-HCl, pH 7.6, 6.6 mM MgCl₂, 10 mM DTT, 10% glycerol and stored at 4°C.

### V. Coupling of Klenow fragment of E. coli DNA Polymerase-I to tresylated CPG-linker-arm.

40 units of Klenow fragment of E. coli DNA Polymerase-I (Bethesda Research Labs.) are dissolved in 200 microliters of buffer consisting of 1.5 mM ATP; 30 mM Tris acetate buffer at pH 7.9; 60 mM sodium acetate; 10 mM magnesium acetate; and added to a lyophilized mixture of 80 microgram Poly (dA)-20 microgram oligo (dT). To this mixture is added 100 milligrams of dry tresylated CPG-linker-arm. The resulting gel slurry is mixed "end-over-end" at 4°C for 10 hours. Residual reactive tresyl groups on the linker-arm are removed by adding 0.1 mM dithiothreitol (DTT), (2 hours, 4C). After coupling, the gel is washed free of uncoupled enzyme by washing (4x0.5 ml) in a buffer consisting of 60 mM sodium acetate, 30 mm Tris acetate, 10 mM magnesium acetate at pH 8.0 and 0.25 mM dithiotreitol.
Finally, the gel is equilibrated in 66 mM Tris-HCl pH 7.6; 6.6 mM magnesium chloride; 10 mM DTT; 10% glycerol and stored at 4°C.

The following examples illustrate the use of these immobilized enzymes in the method of the application.

### EXAMPLE 13

### Step 1: The synthesis of the Chlamydia sequence.

The desired sequence to be amplified using the gap-filling/ligating system of joining the oligonucleotides is a 88 base pair that coded for Chlamydia trachomatis cryptic plasmid. This particular sequence is a four times repeat sequence of 22 base pairs, that has been obtained by the sequencing of the 6.5 kilobase plasmid derived from Chlamydia trachomatis (serotype L) inserted into the unique Bam H1/ SStl sites of the PUC18 plasmid. The 6.5 Kb are sequenced using the dideoxy chain termination method of Sanger et al. P.N.A.S. 83, 1911 (1986).

The 22 repeated base pair has the following sequence:

5'-(GTCTACCACCAAGAGTTGCAAA)x4-3'
3'-(CAGATGGTGGTTCTCAACGTTT)x4-5'

The construction of the two pairs is as follows: 
Y = 5'-AGTCTACCACCAAGAGTTGC-3'
Z = 3'- CAGATGGTGGTTCTCAACGT-5'
Y represents in this case oligonucleotides A and B in the oligonucleotide complement pairs.
Z represents in this case oligonucleotides A' and B' in the oligonucleotide complement pairs. When hybridized to the target sequence, Y and Z form a gap of two complementary base pairs, A-T.

The two oligodeoxyribonucleotides described above are synthesized and purified by the following procedure.

### I. Automated Synthesis Procedures. (according to Matteucci et al., J. Am. Chem. oc., 103, 31-5, (1981).

The 2-cyanoethyl phosphoramidites are purchased from Applied Biosystems Inc. The procedure includes condensation of nucleoside phosphoramidites to a nucleoside-derivatized controlled pore glass bead support, (500Å) 30 mg, using NDA synthesizer from Applied Biosystems Inc., Type 380B-02. The cycles includes detritylation with 2% trichloroacetic acid in dichloromethane; condensation using tetrazole as an activating proton donor; capping with acetic anhydride and dimethylaminopyridine; oxidation of the phosphite to the phosphate with 0.1 M I2/H20/Lutidine/Tetrahydrofuam, following detritylation using 2% trichloroacetic acid in dichloromethane. Cycle time is approximately 30 minutes. Yields at each step are essentially quantitative and are determined by collection and spectropscopic examination of the dimethoxytrityl alcohol released during detritylation.

### II. Oligodeoxyribonucleotide Deprotection and Purification Procedures.

The solid support is removed from the column and exposed to 1 ml concentrated ammonium hydroxide at 60° C for 16 hours in a closed tube. Ammonia is removed and the residue is applied to a preparative 12% polyacrylamide gel using a Tris-borate buffer (pH 8) containing 7M urea. Electrophoresis is carried out at 20 volts/cm for 5 hours after which the band containing the product is identified by UV shadowing of a fluorescent plate. The band is excised and eluted with 1 ml double distilled water overnight at room temperature. This solution is filtered and the supernatant is extracted (3x300 microliter) with n-butanol. The water phase is placed on a Sephadex G-50 column (Pharmacia) (1x10 cm). The elutions are monitored by UV absorbance at 260 nm and the appropriate fraction collected, quantitated by UV absorbance in a fixed volume and evaporated to dryness at room temperature in a vacuum centrifuge. III. 5'-Phosphorylation of A, B, A' and B'.

The oligodeoxyribonucleotides Y and Z (5 nanomoles each) are lyophilized separately to dryness and redissolved in (50 microliter) of 50 nM HEPES (Aldrich) pH 7.6, 10 nM, MgCl₂, 10 mM dithiotreitol and [ ³P]-ATP (10nM).
Specific activity = 2.5 x 10³ cpm/pmole.
Each tube is incubated with two units of T₄-polynucleotide kinase for 40 minutes at 37°C for five minutes to inactive the enzyme, and are loaded on Sephadex columns G50 and eluted with water. The elutions are monitored by gamma counter (Beckman) and lyophilized to dryness.

### EXAMPLE 14

### I. Purification of Chlamydial DNA

Chlamydia trachomatis LGV2 strain is grown in McCoy cells in the presence of 1 microgram cycloheximide/ml until about 1.0 IFU (inclusion-forming unit) per cell. The cells are grown for 3 days at which time 80-90% of them showed visible inclusions and are harvested and stored at -70°C until tested. Control microbial strains are obtained from routine isolations (Department of Microbiology, Ben-Gurion University) and identified by standard procedures (Lennette et al., Manual of Clinical Microbiology, 3rd edition, Washington (1980)). The strains include isolates of E. coli and Streptococcus agalactiae.

The microbial strains are grown on plates or in liquid medium. For the test, a suspension of each of the microbes is boiled at an alkaline pH (0.3 M NaOH), chilled on ice and neutralized with HCl.

The Chlamydial cell culture harvests of 100 ml are disrupted with a glass-Teflon homogenizer and centrifuged at 1.400 x g for 5 minutes to remove cell debris. The supernatant is layered onto 30% (vol/vol) Renografin -76 (E.R. Squibb, Princeton U.S.A.) diluted with 20 mM Tris-150 mM HCl (THCl) (pH 7.5); Renografin -76, (a solution of diatrizoate meglumine and diatrizoate sodium in citrate buffer). After centrifugation at 50,000 x g for 45 minutes, the pellets are resuspended in 1.0 ml of THCl, layered onto 30 to 60% (vol/vol), Renografin -76 gradients in THCl, and centrifuged at 50.000 x g for 2 hr. Two diffuse bands are visible in the middle region of the gradient; these fractions are collected, diluted with THCl and centrifuged at 50.000 x g for 45 min. The pellets are resuspended in 0.5 ml of THCl. II. DNA Extraction

The following procedure is based on the method described by (Wenman et al., Nature 296:68-70 (1982)).

The Chlamydial suspension is homogenized before the addition of 50 mM Tris containing 20 mM Na₂ EDTA 20% (wt/vol) sucrose, 0.7% (wt/vol) N-lauroylsarcosine; and 200 microgram of proteinase K. This mixture is incubated at 55°C for 15 min. and then at 37°C for 45 min. An equal volume of phenolchloroform is added and the mixture is vortexed and centrifuged at 2,000 x g for 10 min. Nucleic acids are precipitated from the aqueous phase by addition of a 1/20 volume of 5M NaCl and 2 volumes of ethanol. After two further cycles of centrifugation and precipitation, the DNA is measured spectrophotometrically, precipitated once more, and redissolved in 10 mM Tris with 1 mM EDTA (pH 7.0) at a concentration of 200 microgram/ml.

DNA is digested in 4 to 10 microgram amounts at a concentration of 80 microgram/ml with 4 units of BAM-H1 (B1) per microgram of DNA for 2 to 3 hours, under the conditions recommended by the manufacturer.

### EXAMPLE 15

This example illustrates the gap filling-ligation amplification steps using immobilized enzymes employing the apparatus as shown in Figure 3.

A mixture of polymeric beads 1, (10 mg beads) of CPG-T₄-DNA ligase from Example 12. IV and (10 mg beads) of CPG-Klenow fragment of E. coli. DNA Polymerase-I from Example 12.V, are placed in plastic cylinder 2 (inner volume ∼ 0.5 ml), where the edges of cylinder 2 contain plastic covers 3a, 3b, having inlet 4 and outlet 5 that connect to plastic tubing 6, 7. The bottom of cylinder 2 is filled with siliconized glass wool 8, which holds beads 1.

Inlet 4 and outlet 5 are attached to plastic tubing 6, 7, the tubing has a diameter of 0.5 mm. Plastic tubing 7 is attached to stainless steel coil 9, which is immersed in hot oil bath 10, containing silicon oil at 90°C. Stainless steel coil 9 is attached by tube 11 to second stainless coil 12. Coil 12 is immersed in a water bath 13, held at room temperature. Coils 9 and 12 are also 0.5 mm in diameter. Coil 12 is attached to plastic tube 14, having a diameter of 0.5 mm. Tube 14 is connected to peristaltic pump 15 and thereafter to cell 2 through inlet 4. The pump pushes the solution through the system at a rate of two drops per minute.

### Step 1. Protection of Y and Z Oligonucleotides from the 3'-5' Exonuclease Activity of DNA Polymerase I and to Form Blunt Ends.

100 picomole of each 5'-phosphorylated Y and Z are mixed together and dissolved in 1 millimeter of 1 mM MgCl₂, 20 mM 2-mercaptoethanol, 100 mg of bovine serum albumin per ml and 50 mM Tris-HCl (pH 7.5), and 0.1 mM of dATP[αS] and TTP[αS](Sigma).

The resulting solution is heated to 100°C for 2 minutes which allows separation of the strands and hybridization of the Y and Z to the target. Then 5 units of Klenow fragment of E. coli DNA polymerase-I are added and the reaction is incubated for 10 minutes at room temperature. The pairs are now blunt-ended and protected against exonuclease activity of polymerase. Step 2. 1 microgram of Bam HI digested DNA from Example 14.II above is dissolved in the buffer resulted from step 1. To this buffer is added 1 nanomole of dATP and TTP and 100 p moles of each of α-³P[dATP] and α-³P[TTP]. Specific activity 1000 cpm/picamole.

This solution is injected through inlet 4 of the apparatus and the cycling is started by operating peristaltic pump 15.

After 3 hours of cycling the solution through cell 2 containing the immobilized enzymes, the solution is loaded on a Sephadex column G-40/50 (1 x 10 cm) and eluted with doubly distilled water. Fractions of 1 ml are collected and monitored by a Geiger counter.

Two distinct bands are detected, the first is eluted in a pool of 4-7 ml containing labeled amplified joined oligonucleotide products by gap filling/ligation process. This pool has about 115000 cpm whereas the second pool that eluted in 14-17 ml has 80,000 cpm.

## Claims

1. A process for detecting the presence or absence of at least one specific nucleic acid target sequence, or a portion thereof, by amplifying the nucleic acid target sequence(s), or portion(s) thereof, in a sample suspected of containing the target sequence(s), comprising the steps of:
a) treating the sample with at least two oligonucleotides for each strand of a target sequence, under hybridizing conditions,
i) wherein the oligonucleotides are selected so as to be sufficiently complementary to each strand of the target sequence(s) or portion(s) thereof to hybridize therewith; and
ii) wherein a gap of one or more bases is present between the oligonucleotides when the oligonucleotides are hybridized to the target sequence(s), or portion(s) thereof; and
iii) wherein the oligonucleotides are selected so that the gaps between them will require less than all four bases to fill in the gap;
b) filling the gap formed in step (a) with one or more bases complementary to the base or bases in the gap and joining the base or bases filling the gap to each other and to both adjacent hybridized oligonucleotides, thereby forming a joined oligonucleotide product;
c) treating the hybridized joined oligonucleotide product of step (b) under denaturing conditions to separate the joined oligonucleotide product(s) from the target sequence(s), or portion(s) thereof, to produce single-stranded molecules;
d) treating the single-stranded molecules produced in step (c) with at least two oligonucleotide complement pairs, under hybridizing conditions,
i) wherein each oligonucleotide complement pair comprises two ologonucleotides selected so as to be sufficiently complementary to each other, to each strand of the target sequence(s), or portion(s) thereof, and to the joined oligonucleotide product(s), or portion(s) thereof, to hybridize therewith; and
ii) wherein a gap of one or more bases is present between the oligonucleotides when the oligonucleotides are hybridized to the target sequence(s), or portion(s) thereof; and
iii) wherein the oligonucleotides are selected so that the gaps between them will require less than all four bases to fill in the gap;
e) filling in the gap formed in step (d) with one or more bases complementary to the base or bases in the gap and joining the bases filling the gap to each other and to both adjacent hybridized oligonucleotides or oligonucleotide complement pairs, thereby forming additional joined oligonucleotide product(s), resulting in the amplification of the target sequences(s) or portion(s) thereof; and
f) detecting the joined oligonucleotide product(s).

2. The process of claim 1, further including step
e') treating the hybridized oligonucleotides of step (e) under denaturing conditions to separate the hybridized oligonucleotides and produce single-stranded molecules, wherein steps (d), (e) and (e') are repeated a desired number of times.

3. The process of claim 1, wherein the nucleic acid target sequence(s), or portion(s) thereof, is double-stranded DNA and its strands are separated before or during step (a).

4. The process of claim 1, wherein the nucleic acid target sequence(s), or portion(s) thereof, is single-stranded DNA or RNA.

5. The process of claim 1, wherein the oligonucleotides are oligodeoxyribonucleotides.

6. The process of claim 1, wherein the oligonucleotide complement pairs are present as a molar excess in the range of 10⁵ to 10¹⁵ pairs per nucleic acid target sequence(s), or portion(s) thereof.

7. The process of claim 1, wherein the gap is filled by catalytic agents.

8. The process of claim 7, wherein the catalytic agents are a combination comprising a DNA polymerase and a DNA ligase.

9. The process of claim 7, wherein the catalytic agents are immobilized on polymeric supports.

10. The process of claim 9, wherein the DNA polymerase is selected from the group consisting of E. coli DNA polymerase-I, Klenow fragments of E. coli DNA polymerase-I, T4 DNA polymerase, reverse transcriptase and the ligase is selected from the group consisting of E. coli DNA ligase or T4 DNA ligase.

11. The process of claim 9, wherein the polymerase and ligase are heat stable.

12. The process of claim 11, wherein the heat stable polymerase and ligase are isolated from a thermophilic bacteria.

13. The process of claim 7, wherein deoxyribonucleotide triphosphates are added to the sample mixture in steps (a) and (d).

14. The process of claim 13, wherein the oligonucleotides and /or deoxyribonucleic triphosphates are modified to be resistant to 3'--> 5' exonuclease activity.

15. A process for amplifying at least one specific nucleic acid target sequence, or a portion thereof, in a sample containing a nucleic acid or a mixture of nucleic acids, comprising the steps of:
a) treating the sample with at least two oligonucleotides for each strand of a target sequence, under hybridizing conditions,
i) wherein the oligonucleotides are selected so as to be sufficiently complementary to each strand of the target sequence(s), or portion(s) thereof to hybridize therewith; and
ii) wherein a gap of one or more bases is present between the oligonucleotides when the oligonucleotides are hybridized to the target sequence(s), or portion(s) thereof; and
iii) wherein the oligonucleotides are selected so that the gaps between them will require less than all four bases to fill the gaps;
b) filling the gap formed in step (a) with one or more bases complementary to the base or bases in the gap and joining the bases filling the gap to each other and to both adjacent hybridized oligonucleotides, thereby forming a joined olignucleotide products;
c) treating the hybridized oligonucleotides of step (b) under denaturing conditions to separate the joined oligonucleotide product(s) from the target sequence(s) or portion(s) thereof, to produce single-stranded molecules;
d) treating the single-stranded molecules produced in step (c) with at least two oligonucleotide complement pairs,
i) wherein each oligonucleotide complement pair comprises two oligonucleotides selected so as to be sufficiently complementary to each other, to each strand of the target sequence(s), or portion(s) thereof, and to the joined oligonucleotide product(s) or portion(s) thereof, to hybridize therewith; and
ii) wherein a gap of one or more bases is present between the oligonucleotides when the oligonucleotides are hybridized to the target sequence(s), or portion(s) thereof; and
iii) wherein the oligonucleotides are selected so that the gaps between them will require less than all four bases to fill in the gap;
e) filling the gap formed in step (d) with one or more bases complementary to the base or bases in the gap and joining the base filling the gap to each other and to both adjacent hybridized oligonucleotides or oligonucleotide complement pairs, thereby forming additional joined oligonucleotide product(s), resulting in the amplification of the target sequence(s), or portion(s) thereof.

16. The process of claim 15 further including step
(e') treating the hybridized oligonucleotides of step (e) under denaturing conditions to separate the hybridized oligonucleotides and produce single-stranded molecules, wherein steps (d), (e) and (e') are repeated a desired number of times.

17. The process of claim 15 wherein the nucleic acid target sequence(s), or portion(s) thereof, is double-stranded DNA and its strands are separated before or during step (a).

18. The process of claim 15 wherein the nucleic acid target sequence(s), or portion(s) thereof, is single-stranded DNA or RNA.

19. The process of claim 15 wherein the oligonucleotides are oligodeoxyribonucleotides.

20. The process of claim 15 wherein the oligonucleotide complement pairs are present as a molar excess in the range of 10⁵ to 10¹⁵ pairs per nucleic acid target sequence(s), or portion(s) thereof.

21. The process of claim 15 wherein the gap is filled by catalytic agents.

22. The process of claim 21 wherein the catalytic agents are a combination comprising a DNA polymerase and a DNA ligase.

23. The process of claim 21 wherein the catalytic agents are immobilized on polymeric supports.

24. The process of claim 22 wherein the DNA polymerase is selected from the group consisting of E. coli DNA polymerase-I, Klenow fragments of E. coli DNA polymerase-I, T4 DNA polymerase, reverse transcriptase and the ligase is selected from the group consisting of E. coli DNA ligase or T4 DNA ligase.

25. The process of claim 22 wherein the polymerase and ligase are heat stable.

26. The process of claim 25 wherein the heat stable polymerase and ligase are isolated from a thermophilic bacteria.

27. The process of claim 22 wherein deoxyribonucleotide triphosphates are added to the sample mixture in steps (a) and (d).

28. The process of claim 27 wherein the oligonucleotides and/or deoxyribonucleic triphosphates are modified to be resistant to 3'-->5' exonuclease activity.

29. A kit for detecting the presence or absence of at least one specific nucleic acid target sequence, or portion(s) thereof, in a sample suspected of containing the target sequence(s), according to the process of claim 1 comprising:
at least one container containing at least two oligonucleotide complement pairs, wherein an oligonucleotide complement pair comprises two oligonucleotides selected so as to be sufficiently complementary to each other and to each strand of the target sequence(s), or portion(s) thereof, to hybridize therewith;
a means for mixing the sample with the oligonucleotide complement pairs under hybridizing conditions;
a means for joining an oligonucleotide from a first oligonucleotide complement pair to an oligonucleotide from a second oligonucleotide complement pair, when both oligonucleotides are hybridized to the same strand of a nucleic acid, to form a joined oligonucleotide product, after the sample and oligonucleotide complement pairs are mixed, wherein the means for joining the oligonucleotide from the first oligonucleotide complement pair to the oligonucleotide from the second complement pair comprise a DNA polymerase, a DNA ligase, and deoxyribonucleotide triphosphates;
and a means for detecting the joined oligonucleotide products.

30. The kit of claim 29 further comprising a container containing a positive control for the oligonucleotide complement pairs which contains the specific nucleic acid target squence(s), or portion(s) thereof; and
a container containing a negative control for the oligonucleotide complement pairs which does not contain the specific nucleic acid target sequence(s) or portion(s) thereof.

31. The kit of claim 29 wherein the oligonucleotides are oligodeoxyribonucleotides.

32. The kit of claim 29 wherein the DNA polymerase is selected from the group consisting of E. coli DNA polymerase-I, Klenow fragments of E. coli DNA polymerase-I, T4 DNA polymerase, reverse transcriptase and the ligase is selected from the group consisting of E. coli DNA ligase or T4 DNA ligase.

33. The kit of claim 29 wherein the DNA polymerase and DNA ligase are heat stable.

34. The kit of claim 29 wherein the DNA polymerase and DNA ligase are immobilized on polymeric supports.

35. The kit of claim 29 wherein the deoxyribonucleotide triphosphates are labelled.

36. The kit of claim 29 wherein the means to detect the joined oligonucleotide product detects the labelled deoxyribonucleotide triphosphates.

## Patentansprüche

1. Verfahren zum Nachweisen der Gegenwart oder Abwesenheit von mindestens einer spezifischen Nukleinsäurezielsequenz oder einem Teil davon durch Amplifizierender Nukleinsäurezielsequenz(en) oder einem Teil (Teilen) davon in einer Probe, von der vermutet wird, daß sie die Zielsequenz(en) enthält, umfassend die folgenden Schritte:
(a) Behandeln der Probe mit mindestens zwei Oligonukleotiden für jeden Strang einer Zielsequenz unter Hybridisierungsbedingungen,
(i) wobei die Oligonukleotide so ausgewählt werden, daß sie jedem Strang der Zielsequenz(en) oder einem Teil (Teilen) davon ausreichend komplementär sind, um damit zu hybridisieren; und
(ii) wobei eine Lücke von einer oder mehreren Basen zwischen den Oligonukleotiden vorhanden ist, wenn die Oligonukleotide mit der (den) Zielsequenz(en) oder einem Teil (Teilen) davon hybridisiert werden; und
(iii) wobei die Oligonukleotide so ausgewählt werden, daß das Auffüllen der Lücken zwischen ihnen weniger als alle vier Basen erfordert;
(b) Auffüllen der in Schritt (a) gebildeten Lücke mit einer oder mehreren Basen, die der Base oder den Basen in der Lücke komplementär sind, und Verbinden der Base oder Basen, die die Lücke füllen, miteinander und mit den beiden benachbarten hybridisierten oligonukleotiden, wodurch ein verbundenes Oligonukleotidprodukt gebildet wird;
(c) Behandeln des hybridisierten verbundenen Oligonukleotidproduktes aus Schritt (b) unter denaturierenden Bedingungen, um das verbundene Oligonukleotidprodukt (die verbundenen Oligonukleotidprodukte) von der (den) Zielsequenz(en) oder einem Teil (Teilen) davon zu trennen, um einzelsträngige Moleküle zu erzeugen;
(d) Behandeln der in Schritt (c) erzeugten einzelsträngigen Moleküle mit mindestens zwei Oligonukleotidkomplementpaaren unter hybridisierenden Bedingungen,
(i) wobei jedes Oligonukleotidkomplementpaar zwei Oligonukleotide umfaßt, die so ausgewählt sind, daß sie ausreichend komplementär zueinander, zu jedem Strang der Zielsequenz(en) oder einem Teil (Teilen) davon und zu den verbundenen Oligonukleotidprodukt(en) oder einem Teil (Teilen) davon sind, um damit zu hybridisieren; und
(ii) wobei eine Lücke von einer oder mehreren Basen zwischen den Oligonukleotiden vorhanden ist, wenn die Oligonukleotide mit der (den) Zielsequenz(en) oder einem Teil (Teilen) davon hybridisiert werden; und
(iii) wobei die Oligonukleotide so ausgewählt werden, daß zum Auffüllen der Lücken zwischen ihnen weniger als alle vier Basen erforderlich sind;
(e) Auffüllen der in Schritt (d) gebildeten Lücke mit einer oder mehreren Basen, die der Base oder den Basen in der Lücke komplementär sind, und Verbinden der Basen, die die Lücke füllen, miteinander und mit den beiden benachbarten hybridisierten Oligonukleotiden oder Oligonukleotidkomplementpaaren, wodurch ein zusätzliches verbundenes Oligonukleotidprodukt (Oligonukleotidprodukte) gebildet wird (werden), was zu einer Amplifikation der Zielsequenz(en) oder eines Teiles (von Teilen) davon führt; und
(f) Nachweisen des verbundenen Oligonukleotidproduktes (der Oligonukleotidprodukte).

2. Verfahren nach Anspruch 1, weiter umfassend den Schritt
(e') des Behandelns der hybridisierten Oligonukleotide aus Schritt (e) unter denaturierenden Bedingungen, um die hybridisierten Oligonukleotide zu trennen und einzelsträngige Moleküle zu erzeugen, wobei die Schritte (d), (e) und (e') eine gewünschte Anzahl von Malen wiederholt werden.

3. Verfahren nach Anspruch 1, wobei die Nukleotidzielsequenz(en) oder ein Teil (Teile) davon doppelsträngige DNA ist (sind) und ihre Stränge vor oder während Schritt (a) getrennt werden.

4. Verfahren nach Anspruch 1, wobei die Nukleinsäurezielsequenz(en) oder ein Teil (Teile) davon einzelsträngige DNA oder RNA ist (sind).

5. Verfahren nach Anspruch 1, wobei die Oligonukleotide Oligodesoxyribonukleotide sind.

6. Verfahren nach Anspruch 1, wobei Oligonukleotidkomplementpaare in einem molaren Überschuß im Bereich von 10⁵ bis 10¹⁵ Paare pro Nukleinsäurezielsequenz(en) oder einem Teil (Teilen) davon vorhanden sind.

7. Verfahren nach Anspruch 1, wobei die Lücke durch katalytische Mittel gefüllt wird.

8. Verfahren nach Anspruch 7, wobei die katalytischen Mittel eine Kombination sind, die eine DNA-Polymerase und eine DNA-Ligase umfassen.

9. Verfahren nach Anspruch 7, wobei die katalytischen Mittel auf polymeren Trägern immobilisiert sind.

10. Verfahren nach Anspruch 9, wobei die DNA-Polymerase aus der Gruppe ausgewählt ist, die aus E. coli-DNA-Polymerase-I, Klenow-Fragmenten von E. coli-DNA-Polymerase-I, T4-DNA-Polymerase, reverser Transkriptase besteht und die Ligase aus der Gruppe ausgewählt ist, die aus E. coli-DNA-Ligase oder T4-DNA-Ligase besteht.

11. Verfahren nach Anspruch 9, wobei die Polymerase und Ligase wärmestabil sind.

12. Verfahren nach Anspruch 11, wobei die wärmestabile Polymerase und Ligase aus einem thermophilen Bakterium isoliert werden.

13. Verfahren nach Anspruch 7, wobei der Probenmischung in den Schritten (a) und (d) Desoxyribonukleotidtriphosphate zugefügt werden.

14. Verfahren nach Anspruch 13, wobei die Oligonukleotide und/oder Desoxyribonukleotidtriphosphate modifiziert sind, um gegen 3' → 5'-Exonukleaseaktivität resistent zu sein.

15. Verfahren zum Amplifizieren mindestens einer spezifischen Nukleinsäurezielsequenz oder eines Teiles davon in einer Probe, die eine Nukleinsäure oder eine Mischung von Nukleinsäuren enthält, umfassend die folgenden Schritte:
(a) Behandeln der Probe mit mindestens zwei Oligonukleotiden für jeden Strang einer Zielsequenz unter Hybridisierungsbedingungen,
(i) wobei die Oligonukleotide so ausgewählt werden, daß sie jedem Strang der Zielsequenz(en) oder einem Teil (Teilen) davon ausreichend komplementär sind, um damit zu hybridisieren; und
(ii) wobei eine Lücke von einer oder mehreren Basen zwischen den Oligonukleotiden vorhanden ist, wenn die Oligonukleotide mit der (den) Zielsequenz(en) oder einem Teil (Teilen) davon hybridisiert werden; und
(iii) wobei die Oligonukleotide so ausgewählt werden, daß das Auffüllen der Lücken zwischen ihnen weniger als alle vier Basen erfordert;
(b) Auffüllen der in Schritt (a) gebildeten Lücke mit einer oder mehreren Basen, die der Base oder den Basen in der Lücke komplementär sind, und Verbinden der Base oder Basen, die die Lücke füllen, miteinander und mit den beiden benachbarten hybridisierten Oligonukleotiden, wodurch ein verbundenes Oligonukleotidprodukt gebildet wird;
(c) Behandeln der hybridisierten Oligonukleotidprodukte aus Schritt (b) unter denaturierenden Bedingungen, um das verbundene Oligonukleotidprodukt (die verbundenen Oligonukleotidprodukte) von der (den) Zielsequenz(en) oder einem Teil (Teilen) davon zu trennen, um einzelsträngige Moleküle zu erzeugen;
(d) Behandeln der in Schritt (c) erzeugten einzelsträngigen Moleküle mit mindestens zwei Oligonukleotidkomplementpaaren,
(i) wobei jedes Oligonukleotidkomplementpaar zwei Oligonukleotide umfaßt, die so ausgewählt sind, daß sie ausreichend komplementär zueinander, zu jedem Strang der Zielsequenz(en) oder einem Teil (Teilen) davon und zu den verbundenen Oligonukleotidprodukt(en) oder einem Teil (Teilen) davon sind, um damit zu hybridisieren; und
(ii) wobei eine Lücke von einer oder mehreren Basen zwischen den Oligonukleotiden vorhanden ist, wenn die Oligonukleotide mit der (den) Zielsequenz(en) oder einem Teil (Teilen) davon hybridisiert werden; und
(iii) wobei die Oligonukleotide so ausgewählt werden, daß zum Auffüllen der Lücken zwischen ihnen weniger als alle vier Basen erforderlich sind;
(e) Auffüllen der in Schritt (d) gebildeten Lücke mit einer oder mehreren Basen, die der Base oder den Basen in der Lücke komplementär sind, und Verbinden der Basen, die die Lücke füllen, miteinander und mit den beiden benachbarten hybridisierten Oligonukleotiden oder Oligonukleotidkomplementpaaren, wodurch ein zusätzliches verbundenes Oligonukleotidprodukt (Oligonukleotidprodukte) gebildet wird (werden), was zu einer Amplifikation der Zielsequenz(en) oder eines Teiles (von Teilen) davon führt.

16. Verfahren nach Anspruch 15, weiter umfassend den Schritt
(e') des Behandelns der hybridisierten Oligonukleotide aus Schritt (e) unter denaturierenden Bedingungen, um die hybridisierten Oligonukleotide zu trennen und einzelsträngige Moleküle zu erzeugen, wobei die Schritte (d), (e) und (e') eine gewünschte Anzahl von Malen wiederholt werden.

17. Verfahren nach Anspruch 15, wobei die Nukleinsäurezielsequenz(en) oder ein Teil (Teile) davon doppelsträngige DNA ist (sind) und ihre Stränge vor oder während Schritt (a) getrennt werden.

18. Verfahren nach Anspruch 15, wobei die Nukleinsäurezielsequenz(en) oder ein Teil (Teile) davon einzelsträngige DNA oder RNA ist (sind).

19. Verfahren nach Anspruch 15, wobei die Oligonukleotide Oligodesoxyribonukleotide sind.

20. Verfahren nach Anspruch 15, wobei Oligonukleotidkomplementpaare als molarer Überschuß im Bereich von 10⁵ bis 10¹⁵ Paaren pro Nukleinsäurezielsequenz(en) oder einem Teil (Teilen) davon vorliegen.

21. Verfahren nach Anspruch 15, wobei die Lücke durch katalytische Mittel gefüllt wird.

22. Verfahren nach Anspruch 21, wobei die katalytischen Mittel eine Kombination sind, die eine DNA-Polymerase und eine DNA-Ligase umfassen.

23. Verfahren nach Anspruch 21, wobei die katalytischen Mittel auf polymeren Trägern immobilisiert sind.

24. Verfahren nach Anspruch 22, wobei die DNA-Polymerase aus der Gruppe ausgewählt ist, die aus E. coli-DNA-Polymerase-I, Klenow-Fragmenten von E. coli-DNA-Polymerase-I, T4-DNA-Polymerase, reverser Transkriptase besteht und die Ligase aus der Gruppe ausgewählt ist, die aus E. coli-DNA-Ligase und T4-DNA-Ligase besteht.

25. Verfahren nach Anspruch 22, wobei die Polymerase und Ligase wärmestabil sind.

26. Verfahren nach Anspruch 25, wobei die wärmestabile Polymerase und Ligase aus einem thermophilen Bakterium isoliert werden.

27. Verfahren nach Anspruch 22, wobei der Probenmischung in den Schritten (a) und (d) Desoxyribonukleotidtriphosphate zugefügt werden.

28. Verfahren nach Anspruch 27, wobei die Oligonukleotide und/oder Desoxyribonukleotidtriphosphate modifiziert sind, um gegen 3' → 5'-Exonukleaseaktivität resistent zu sein.

29. Kit zum Nachweisen der Gegenwart oder Abwesenheit von mindestens einer spezifischen Nukleinsäurezielsequenz oder einem Teil (Teilen) davon in einer Probe, von der vermutet wird, daß sie die Zielsequenz(en) enthält, gemäß dem Verfahren von Anspruch 1, umfassend:
mindestens einen Behälter, der mindestens zwei Oligonukleotidkomplementpaare enthält, wobei ein Oligonukleotidkomplementpaar zwei Oligonukleotide umfaßt, die so ausgewählt sind, daß sie ausreichend komplementär zueinander und zu jedem Strang der Zielsequenz(en) oder einem Teil (Teilen) davon sind, um damit zu hybridisieren;
ein Mittel zum Mischen der Probe mit den Oligonukleotidkomplementpaaren unter Hybridisierungsbedingungen;
ein Mittel zum Verbinden eines Oligonukleotides von einem ersten Oligonukleotidkomplementpaar mit einem Oligonukleotid von einem zweiten Oligonukleotidkomplementpaar, wenn beide Oligonukleotide an den gleichen Strang einer Nukleinsäure hybridisiert sind, um ein verbundenes Oligonukleotidprodukt zu bilden, nachdem die Probe und die Oligonukleotidkomplementpaare gemischt sind, wobei das Mittel zum Verbinden des Oligonukleotids aus dem ersten Oligonukleotidkomplementpaar mit dem Oligonukleotid aus dem zweiten Komplementpaar eine DNA-Polymerase, eine DNA-Ligase und Desoxyribonukleotidtriphosphate umfaßt;
und ein Mittel zum Nachweisen der verbundenen Oligonukleotidprodukte.

30. Kit nach Anspruch 29, weiter umfassend einen Behälter, der eine positive Kontrolle für die Oligonukleotidkomplementpaare enthält, die die spezifischen Nukleinsäurezielsequenz(en) oder einen Teil (Teile) davon enthält; und
einen Behälter, der eine negative Kontrolle für die Oligonukleotidkomplementpaare enthält, die nicht die spezifischen Nukleinsäurezielsequenz(en) oder einen Teil (Teile) davon enthält.

31. Kit nach Anspruch 29, wobei die Oligonukleotide Oligodesoxyribonukleotide sind.

32. Kit nach Anspruch 29, wobei die DNA-Polymerase aus der Gruppe ausgewählt ist, die aus E. coli-DNA-Polymerase-I, Klenow-Fragmenten von E. coli-DNA-Polymerase-I, T4-DNA-Polymerase, reverser Transkriptase besteht und die DNA-Ligase ausgewählt ist aus der Gruppe, die aus E. coli-DNA-Ligase und T4-DNA-Ligase besteht.

33. Kit nach Anspruch 29, wobei die DNA-Polymerase und DNA-Ligase wärmestabil sind.

34. Kit nach Anspruch 29, wobei die DNA-Polymerase und DNA-Ligase auf polymeren Trägern immobilisiert sind.

35. Kit nach Anspruch 29, wobei die Desoxyribonukleotidtriphosphate markiert sind.

36. Kit nach Anspruch 29, wobei das Mittel zum Nachweisen des verbundenen Oligonukleotidproduktes die markierten Desoxyribonukleotidtriphosphate nachweist.

## Revendications

1. Procédé de détection de la présence ou de l'absence d'au moins une séquence cible d'acide nucléique spécifique, ou d'une partie de celle-ci, en amplifiant la (les) séquence(s) cible(s) d'acide nucléique, ou de partie(s) de celle(s)-ci, dans un échantillon suspecté de contenir la (les) séquence(s) cible(s), comprenant les étapes de :
a) traitement de l'échantillon avec au moins deux oligonucléotides pour chaque brin d'une séquence cible, dans des conditions d'hybridation,
i) dans lequel les oligonucléotides sont choisis de façon à être suffisamment complémentaires avec chaque brin de la (des) séquence(s) cible(s) ou de partie(s) de celle(s)-ci afin de s'hybrider avec eux, et
ii) dans lequel une brèche correspondant à une ou plusieurs base(s) est présente entre les oligonucléotides lorsque les oligonucléotides s'hybrident sur la (les) séquence(s) cible(s), ou une (des) partie(s) de celle(s)-ci ; et
iii) dans lequel les oligonucléotides sont choisis de façon à ce que les brèches entre eux nécessitent moins que les quatre bases entières pour remplir la brèche ;
b) remplissage de la brèche formée à l'étape (a) avec une ou plusieurs base(s) complémentaire(s) à la base ou aux bases dans la brèche et jonction de la base ou des bases qui remplissent la brèche l'(les) une(s) avec l'(les) autre(s) et avec les deux oligonucléotides hybridés adjacents, et de cette façon, formation d'un produit oligonucléotidique lié ;
c) traitement du produit oligonucléotidique lié hybridé de l'étape (b) dans des conditions dénaturantes afin de séparer le(s) produit(s) oligonucléotidique(s) lié(s) de la (des) séquence(s) cible(s), ou de(s) partie(s) de celle(s)-ci, afin de produire des molécules à simple brin ;
d) traitement des molécules à simple brin produites à l'étape (c) avec au moins deux paires complémentaires d'oligonucléotides, dans des conditions d'hybridation,
i) dans lequel chaque paire complémentaire d'oligonucléotides comprend deux oligonucléotides choisis de façon à ce qu'ils soient suffisamment complémentaires l'un avec l'autre, pour chaque brin de la (des) séquence(s) cible(s), ou de(s) partie(s) de celle(s)-ci et pour le(s) produit(s) oligonucléotidique(s) lié(s), ou de(s) partie(s) de celui-ci (ceux-ci), afin de s'hybrider avec ceux-ci ; et
ii) dans lequel une brèche d'une ou plusieurs bases est présente entre les oligonucléotides lorsque les oligonucléotides sont hybridés à la (aux) séquence(s) cible(s), ou à une (des) partie(s) de celle(s)-ci ; et
iii) dans lequel les oligonucléotides sont choisis de façon à ce que les brèches entre eux nécessitent moins que les quatre bases entières pour remplir la brèche ;
e) remplissage de la brèche formée à l'étape (d) avec une ou plusieurs bases complémentaires à la base ou aux bases dans la brèche et jonction des bases remplissant la brèche les unes avec les autres et avec les deux oligonucléotides hybridés adjacents ou les paires complémentaires d'oligonucléotides, et de cette façon formation d'un (de) produit(s) oligonucléotidique(s) lié(s) supplémentaire(s), ce qui entraînera l'amplification de la (des) séquence(s) cible(s) ou de partie(s) de celle(s)-ci ; et
f) détection du (des) produit(s) oligonucléotidique(s) lié(s).

2. Procédé de la revendication 1, incluant, de plus, l'étape de :
e') traitement des oligonucléotides hybridés de l'étape (e) dans des conditions dénaturantes pour séparer les oligonucléotides hybridés et produire des molécules à simple brin, dans lequel les étapes (d), (e) et (e') sont répétées un nombre de fois désiré.

3. Procédé de la revendication 1, dans lequel la (les) séquence(s) cible(s) d'acide nucléique ou une (des) partie(s) de celle(s)-ci est de l'ADN à double brin, et ses brins sont séparés avant l'étape (a) ou pendant celle-ci.

4. Procédé de la revendication 1, dans lequel la (les) séquence(s) cible(s) d'acide nucléique ou une (des) partie(s) de celle(s)-ci est (sont) de l'ADN ou de l'ARN à un seul brin.

5. Procédé de la revendication 1, dans lequel les oligonucléotides sont des oligodésoxyribonucléotides.

6. Procédé de la revendication 1 dans lequel les paires complémentaires d'oligonucléotides sont présentes en tant qu'excès molaire dans une fourchette de 10⁵ à 10¹⁵ paires par séquence(s) cible(s) d'acides nucléiques ou partie(s) de celle-ci.

7. Procédé de la revendication 1, dans lequel la brèche est remplie au moyen d'agents catalytiques.

8. Procédé de la revendication 7, dans lequel les agents catalytiques sont une combinaison comprenant une ADN-polymérase et une ADN-ligase.

9. Procédé de la revendication 7, dans lequel les agents catalytiques sont immobilisés sur des supports polymères.

10. Procédé de la revendication 9, dans lequel l'ADN-polymérase est choisie parmi un groupe consistant en ADN polymérase-I d'E.coli, fragments de Klenow, d'ADN polymérase-I d'E.coli, ADN-polymérase T4, transcriptase inverse et la ligase est choisie parmi un groupe consistant en une ADN-ligase d'E.coli ou une ADN-ligase T4.

11. Procédé de la revendication 9, dans lequel la polymérase et la ligase sont stables à la chaleur.

12. Procédé de la revendication 11, dans lequel les polymérase et ligase stables à la chaleur sont isolées d'une bactérie thermophile.

13. Procédé de la revendication 7 dans lequel des triphosphates désoxyribonucléotidiques sont ajoutés au mélange d'échantillon aux étapes (a) et (d).

14. Procédé de la revendication 13, dans lequel les oligonucléotides et/ou les triphosphates désoxyribonucléiques sont modifiés pour devenir résistants à une activité d'exonucléase de 3' --> 5'.

15. Procédé d'amplification d'au moins une séquence cible d'acide nucléique spécifique, ou d'une partie de celle-ci, dans un échantillon contenant un acide nucléique ou un mélange d'acides nucléiques, comprenant les étapes de :
a) traitement de l'échantillon avec au moins deux oligonucléotides pour chaque brin d'une séquence cible, dans des conditions d'hybridation,
i) dans lequel les oligonucléotides sont choisis de façon à être suffisamment complémentaires avec chaque brin de la (des) séquence(s) cible(s) ou de partie(s) de celle(s)-ci afin de s'hybrider avec eux ; et
ii) dans lequel une brèche d'une ou plusieurs base(s) est présente entre les oligonucléotides lorsque les oligonucléotides s'hybrident sur la (les) séquence(s) cible(s), ou une (des) partie(s) de celle(s)-ci ; et
iii) dans lequel les oligonucléotides sont choisis de façon à ce que les brèches entre eux nécessitent moins que les quatre bases entières pour remplir la brèche ;
b) remplissage de la brèche formée à l'étape (a) avec une ou plusieurs base(s) complémentaire(s) à la base ou aux bases dans la brèche et jonction des bases qui remplissent la brèche les unes avec les autres et avec les deux oligonucléotides hybridés adjacents, et de cette façon, formation d'un produit oligonucléotidique lié ;
c) traitement des oligonucléotides hybridés de l'étape (b) dans des conditions dénaturantes afin de séparer le(s) produit(s) oligonucléotidique(s) lié(s) de la (des) séquence(s) cible(s), ou de(s) partie(s) de celle(s)-ci, afin de produire des molécules à simple brin ;
d) traitement des molécules à simple brin produites à l'étape (c) avec au moins deux paires complémentaires d'oligonucléotides.
i) dans lequel chaque paire complémentaire d'oligonucléotides comprend deux oligonucléotides choisis de façon à ce qu'ils soient suffisamment complémentaires l'un avec l'autre, avec chaque brin de la (des) séquence(s) cible(s), ou de(s) partie(s) de celle(s)-ci et avec le(s) produit(s) oligonucléotidique(s) lié(s), ou de(s) partie(s) de celui-ci (ceux-ci), afin de s'hybrider avec ceux-ci ; et
ii) dans lequel une brèche d'une ou plusieurs bases est présente entre les oligonucléotides lorsque les oligonucléotides sont hybridés à la (aux) séquence(s) cible(s), ou à une (des) partie(s) de celle(s)-ci ; et
iii) dans lequel les oligonucléotides sont choisis de façon à ce que les brèches entre eux nécessitent moins que les quatre bases entières pour remplir la brèche ;
e) remplissage de la brèche formée à l'étape (d) avec une ou plusieurs bases complémentaires à la base ou aux bases dans la brèche et jonction des bases remplissant la brèche les unes avec les autres et avec les deux oligonucléotides hybridés adjacents ou les paires complémentaires d'oligonucléotides, et de cette façon formation d'un (des) produit(s) oligonucléotidique(s) lié(s) supplémentaire(s), ce qui entraînera l'amplification de la (des) séquence(s) cible(s) ou de partie(s) de celle(s)-ci.

16. Procédé de la revendication 15, incluant, de plus, l'étape de :
(e') traitement des oligonucléotides hybridés de l'étape (e) dans des conditions dénaturantes pour séparer les oligonucléotides hybridés et produire des molécules à simple brin, dans lequel les étapes (d), (e) et (e') sont répétées un nombre de fois désiré.

17. Procédé de la revendication 15, dans lequel la (les) séquence(s) cible(s) d'acide nucléique ou une (des) partie(s) de celle(s)-ci est de l'ADN à double brin, et ses brins sont séparés avant l'étape (a) ou pendant celle-ci.

18. Procédé de la revendication 15, dans lequel la (les) séquence(s) cible(s) d'acides nucléiques ou une (des) partie(s) de celle(s)-ci est de l'ADN ou de l'ARN à simple brin.

19. Procédé de la revendication 15, dans lequel les oligonucléotides sont des oligodésoxyribonucléotides.

20. Procédé de la revendication 15 dans lequel les paires complémentaires d'oligonucléotides sont présentes en tant qu'excès molaire dans une fourchette de 10⁵ à 10¹⁵ paires par séquence (s) cible(s) d'acides nucléiques ou partie(s) de celle-ci.

21. Procédé de la revendication 15, dans lequel la brèche est remplie au moyen d'agents catalytiques.

22. Procédé de la revendication 21, dans lequel les agents catalytiques sont une combinaison comprenant une ADN-polymérase et une ADN-ligase.

23. Procédé de la revendication 21, dans lequel les agents catalytiques sont immobilisés sur des supports polymères.

24. Procédé de la revendication 22 dans lequel l'ADN-polymérase est choisie parmi un groupe consistant en ADN-polymérase-I d'E.coli, fragments de Klenow, d'ADN-polymérase-I d'E.coli, ADN-polymérase T4, transcriptase inverse et la ligase est choisie parmi un groupe consistant en une ADN-ligase d'E. Coli ou une ADN-ligase T4.

25. Procédé de la revendication 22, dans lequel la polymérase et la ligase sont stables à la chaleur.

26. Procédé de la revendication 25, dans lequel les polymérase et ligase stables à la chaleur sont isolées d'une bactérie thermophile.

27. Procédé de la revendication 22 dans lequel des triphosphates désoxyribonucléotidiques sont ajoutés au mélange d'échantillon aux étapes (a) et (d).

28. Procédé de la revendication 27, dans lequel les oligonucléotides et/ou les triphosphates déoxyribonucléiques sont modifiés pour devenir résistants à une activité d'exonucléase de 3' --> 5'

29. Trousse pour la détection de la présence ou de l'absence d'au moins une séquence cible d'acide nucléique spécifique, ou de partie(s) de celle(s)-ci, dans un échantillon suspecté de contenir la (les) séquence(s) cible(s), selon le processus de la revendication 1, comprenant :
au moins un conteneur contenant au moins deux paires complémentaires d'oligonucléotides, dans lequel une paire complémentaire d'oligonucléotides comprend deux oligonucléotides choisis de façon à être suffisamment complémentaires l'un avec à l'autre et avec chaque brin de la (les) séquence(s), ou de partie(s) de celle(s)-ci, afin de s'hybrider avec eux ;
un moyen pour mélanger l'échantillon avec les paires complémentaires d'oligonucléotides dans des conditions d'hybridation ;
un moyen pour joindre un oligonucléotide provenant d'une première paire complémentaire d'oligonucléotides à un oligonucléotide provenant d'une seconde paire complémentaire d'oligonucléotides, lorsque les deux oligonucléotides sont hybridés au même brin d'un acide nucléique, pour former un produit oligonucléotidique lié après que l'échantillon et que les paires olignonucléotides de complément se sont mélangés, dans lequel le moyen de jonction de l'oligonucléotide provenant de la première paire complémentaire d'oligonucléotides à l'oligonucléotide de la seconde paire complémentaire d'oligonucléotides comprend une ADN-polymérase, une ADN-ligase, et des triphosphates désoxyribonucléotidiques ;
et un moyen pour la détection de la jonction des produits oligonucléotidiques liés.

30. Trousse de la revendication 29 comprenant, de plus, un conteneur contenant un contrôle positif pour les paires complémentaires d'oligonucléotides contenant la (les) séquence(s) cible(s) d'acide nucléique spécifique(s), ou une (des) partie(s) de celle(s)-ci ; et
un conteneur contenant un contrôle négatif pour les paires complémentaires d'oligonucléotides ne contenant pas la (les) séquence(s) cible(s) d'acide nucléique spécifique(s) ou de partie(s) de celle-ci.

31. Trousse de la revendication 29, dans lequel les oligonucléotides sont des oligodésoxyribonucléotides.

32. Trousse de la revendication 29, dans lequel l'ADN-polymérase est choisie parmi un groupe consistant en ADN-polymérase-I d'E.coli, fragments de Klenow, d'ADN-polymérase-I d'E.coli, ADN-polymérase T4, transcriptase inverse et la ligase est choisie parmi un groupe consistant en une ADN-ligase d'E. Coli ou une ADN-ligase T4.

33. Trousse de la revendication 29, dans lequel l'ADN polymérase et l'ADN ligase sont stables à la chaleur.

34. Trousse de la revendication 29, dans lequel les ADN polymérase et ligase stables à la chaleur sont immobilisées sur des supports polymères.

35. Trousse de la revendication 29, dans lequel les triphosphates désoxyribonucléotidiques sont marqués.

36. Trousse de la revendication 29, dans lequel le moyen de détection du produit oligonucléotidique lié détecte les triphosphates désoxyribonucléotidiques marqués.
